# EUROPEAN PATENT SPECIFICATION

(11) **EP 2 403 525 B1**
(45) Date of publication and mention of the grant of the patent: **27.09.2017**
(21) Application number: 10749113.6
(22) Date of filing: 24.02.2010
(51) Int. Cl.: A61K 39/112, C12N 1/21, C12N 15/31, C12R 1/42

(54) **ATTENUATED SALMONELLA ENTERICA SEROVAR PARATYPHI A AND USES THEREOF**
GEDÄMPFTE ENTERISCHE SALMONELLEN-PARATYPHI A UND VERWENDUNGEN DAVON
SALMONELLA ENTERICA SEROVAR PARATYPHI A ATTÉNUÉE ET SES UTILISATIONS

(30) Priority: 06.03.2009 US 399091
(43) Date of publication of application: 11.01.2012
(73) Proprietor: University of Maryland, Baltimore, Baltimore, MD 21201 (US)
(72) Inventor: VINDURAMPULLE, Christofer, Thornbury Victoria 3071 (AU); BARRY, Eileen, M., Ellicott City Maryland 21046 (US); LEVINE, Myron, M., Columbia Maryland 21044 (US); GALEN, James, Sykesville Maryland 21784 (US)
(74) Representative: Owen, Deborah Jane
(86) International application number: PCT/US2010/025221
(87) International publication number: WO 2010/101750

(56) References cited:
- WO-A1-98/26799
- WO-A1-2005/042564
- WO-A1-2007/125535
- WO-A2-2007/053489
- US-A- 6 130 082
- KOMORIYA, K. ET AL.: 'Flagellar proteins and type III-exported virulence factors are the predominant proteins secreted into the culture media of Salmonella typhimurium.' MOLECULAR MICROBIOLOGY vol. 34, no. 4, 1999, pages 767 - 779, XP055097424
- BRETT, P.J. ET AL.: 'Structural and Immunological Characterization of Burkholderia pseudomallei 0-Polysaccharide–Flagellin Protein Conjugates.' INFECTION AND IMMUNITY vol. 64, no. 7, 1996, pages 2824 - 2828, XP002421921
- BRETT P J ET AL: "Structural and immunological characterization of Burkholderia pseudomallei O-polysaccharide-flagellin protein conjugates", INFECTION AND IMMUNITY, AMERICAN SOCIETY FOR MICROBIOLOGY, US, vol. 64, no. 7, 1 July 1996 (1996-07-01), pages 2824-2828, XP002421921, ISSN: 0019-9567

## Description

This invention was made with government support under NIH Grant No. AI029471 awarded by the National Institutes of Health. The government has certain rights in the invention.

### BACKGROUND OF THE INVENTION

Enteric fever caused by members of the genus *Salmonella,* including typhoid and paratyphoid fevers, continues to constitute a significant disease and mortality burden among populations in developing countries (Lancet 2005; 366:749-762) and represents a notable risk for travelers (Lancet Infect Dis. 2005; 5(10):623-628). Incidences of enteric fever caused by *Salmonella enterica* serovars Typhi and Paratyphi A (S. Typhi and S. Paratyphi A) are on the rise due to the emergence and spread of antibiotic resistant variants (Lancet Infect Dis. 2005 5(10):623-8). Although the clinical disease caused by S. Paratyphi A is overall somewhat milder than that due to S. Typhi, the former can nevertheless result in full-blown enteric fever with an assortment of complications and, if untreated or improperly treated, can result in death. A need exists for vaccines that are safe and effective in combating *Salmonella* infections.

WO2007/125535 relates to recombinant flagellin from Salmonella for use in a vaccine composition. WO98/26799 relates to a conjugate vaccine comprising O polysaccharides from S. Paratyphi A bound to a carrier. WO 2007/053489 relates to attenuated strains of S. Paratyphi A. Brett & Woods (Infect. Immun., 1996, 64, pp 2824-8) relates to structural and immunological characterization of *Burkholderia pseudomallei* O-polysaccharide-flagellin protein conjugates.

### SUMMARY OF THE INVENTION

Embodiments of the present invention are as defined in the claims. In particular, the present invention relates to a method for preparing a conjugate vaccine, comprising:
(a) mutating a strain of *Salmonella* Paratyphi A to generate an attenuated strain of *Salmonella* Paratyphi A, wherein said attenuated *Salmonella* Paratyphi A strain comprises a mutation of the *clpP* gene and one or more further genetic mutations selected from the group consisting of a mutation of the *guaBA* loci, a mutation of the *guaB* gene and a mutation of the *guaA* gene;
(b) isolating an O polysaccharide (OPS) and a flagellin protein from the attenuated strain of (a), wherein said flagellin protein is phase 1 flagellin (fliC); and
(c) conjugating the OPS to the flagellin protein, thereby preparing a conjugate vaccine.

Disclosed herein is an attenuated S. Paratyphi A strain, preferably a live, attenuated S. Paratyphi A strain.

Disclosed herein, the S. Paratyphi A strains for use in the present invention have at least one attenuating mutation selected from the group consisting of attenuating mutations in the *guaBA* loci, the *guaB* gene, the *guaA* gene, the *clpP* gene and the *clpX* gene*.* In preferred embodiments, the *S*. Paratyphi A strain has an attenuating mutation in the *guaB* gene, the *guaA* gene and the *clpP* gene. Disclosed herein, the *Salmonella* Paratyphi A strain has an attenuating mutation in the *guaB* gene, the *guaA* gene and the *clpX* gene*.* In a further preferred embodiment, the *Salmonella* Paratyphi A strain has an attenuating mutation in the *guaB* gene, the *guaA* gene, the *clpP* gene and the *clpX* gene*.*

In one embodiment the attenuating mutations of the *guaBA* loci, the *guaB* gene, the *guaA* gene, the *clpP* gene, and the *clpX* gene are attenuating mutations that reduce the level of expression the loci or the genes, or that block expression of the loci or the genes.

In another embodiment the attenuating mutations of the *guaBA* loci, the *guaB* gene, the *guaA* gene, the *clpP* gene, and the *clpX* gene are attenuating mutations that reduce the activity of a polypeptide encoded by the loci or the genes, or inactivates a polypeptide encoded by the loci or the genes.

In a preferred embodiment, the *Salmonella* Paratyphi A strain is the S. Paratyphi A 9150 strain.

Disclosed herein are *S*. Paratyphi A strains for use in the invention that have at least one attenuating mutation selected from the group consisting of an attenuating mutation in the *guaBA* loci, the *guaB* gene, the *guaA* gene, the *clpP* gene and the *clpX* gene*,* and that further comprises a stabilized plasmid expression system.

The stabilized plasmid expression system may comprise an expression vector having (a) a restricted-copy-number origin of replication cassette, (b) at least one post-segregational killing cassette, (c) at least one partitioning cassette, and (d) an expression cassette.

The restricted-copy-number origin of replication cassette may comprise (i) a nucleotide sequence encoding an origin of replication that limits the expression vector to an average plasmid copy number of about 2 to 75 copies per cell, (ii) a first unique restriction enzyme cleavage site located 5' of the nucleotide sequence encoding the origin of replication, and (iii) a second unique restriction enzyme cleavage site located 3' of the nucleotide sequence encoding the origin of replication.

The post-segregational killing cassette may comprise (i) a nucleotide sequence encoding at least one post-segregational killing locus, (ii) a third unique restriction enzyme cleavage site located 5' of the nucleotide sequence encoding the post-segregational killing locus, and (iii) a fourth unique restriction enzyme cleavage site located 3' of the nucleotide sequence encoding the post-segregational killing locus.

The partitioning cassette may comprise (i) a nucleotide sequence encoding at least one partitioning function, (ii) a fifth unique restriction enzyme cleavage site 5' of the nucleotide sequence encoding the partitioning function, and (iii) a sixth unique restriction enzyme cleavage site located 3' of the nucleotide sequence encoding the partitioning function.

The expression cassette may comprise (i) a nucleotide sequence encoding a selected antigen operably linked to a promoter, (ii) a seventh unique restriction enzyme cleavage site located 5' of the nucleotide sequence encoding the selected antigen operably linked to a promoter, and (iii) an eighth unique restriction enzyme cleavage site located 3' of the nucleotide sequence encoding the selected antigen operably linked to a promoter.

The nucleotide sequence encoding the origin of replication may be a nucleotide sequence selected from the group consisting of the *ori*E1 sequence of SEQ ID NO:28, the *ori*101 sequence of SEQ ID NO:30, and the *ori*15A sequence of SEQ ID NO:29.

The nucleotide sequence encoding the post-segregational killing locus may be a nucleotide sequence selected from the group consisting of a nucleotide sequence encoding the *ssb* balanced-lethal system, a nucleotide sequence encoding the *asd* balanced-lethal system, a nucleotide sequence encoding the *phd-doc* proteic system, and a nucleotide sequence encoding the *hok-sok* antisense system. More preferably, the post-segregational killing locus is a nucleotide sequence encoding *ssb* balanced-lethal system selected from the group consisting of the *Shigella flexneri ssb* locus, the *Salmonella* Typhi *ssb* locus, and the *E. coli ssb* locus. Even more preferably, the *ssb* balanced-lethal system is a *ssb* locus comprising a *ssb* inducible promoter, a *ssb* constitutive promoter and a *ssb* coding region of *S. flexneri* 2a strain CVD 1208s set forth in SEQ ID NO:34.

The nucleotide sequence encoding the partitioning function may be a nucleotide sequence selected from the group consisting of the *E. coli parA* locus set forth in SEQ ID NO:31 and the *E. coli* pSC101 *par* locus set forth in SEQ ID NO:32.

The promoter may be an inducible promoter, more preferably an *ompC* promoter, even more preferably the *ompC* promoter set forth in SEQ ID NO:33.

The nucleotide sequence encoding a selected antigen may be a nucleotide sequence encoding a homologous antigen. In another embodiment, the nucleotide sequence encoding a selected antigen is a nucleotide sequence encoding a heterologous antigen.

The nucleotide sequence encoding a selected antigen may be a nucleotide sequence encoding a heterologous antigen selected from the group consisting of a viral antigen, a bacterial antigen, a cancer antigen, and an auto-immune antigen.

Disclosed herein is a pharmaceutical formulation comprising one or more of the attenuated *Salmonella* Paratyphi A strains. Preferably the pharmaceutical formulations are oral pharmaceutical formulations.

Disclosed herein is a method of inducing an immune response in a subject, comprising administering an immunologically-effective amount of a pharmaceutical formulation as disclosed herein to a subject. Preferably, the immune response is a protective immune response.

The immunologically-effective amount of the pharmaceutical formulation contains about 10² cfu to about 10¹⁰ cfu, more preferably about 10⁶ cfu to about 10⁹ cfu, of the attenuated S. Paratyphi A strain within the pharmaceutical formulation.

The immune response may be to *Salmonella* Paratyphi A. The immune response may be to the selected antigen. The immune response may be to both *Salmonella* Paratyphi A and the selected antigen.

The Lambda Red-mediated mutagenesis system may be used to mutate or delete various chromosomal loci and genes from the S. Paratyphi strains of the present invention.

### BRIEF DESCRIPTION OF THE DRAWINGS

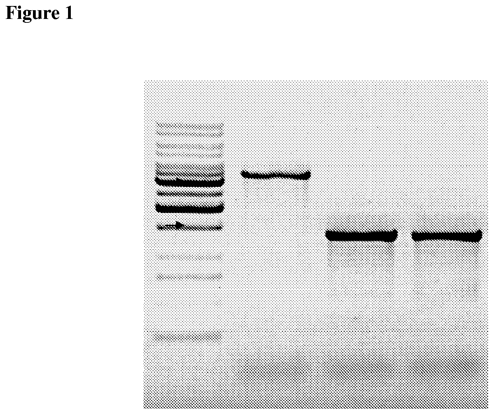

Figure 1 shows the PCR amplification products of *guaBA* and *guaBA::cml.* Lane 1 is wild-type *guaBA*; lanes 2 and 3 are *guaBA*::cml*.* Arrows indicate molecular weight marker bands of 3 kb (top) and 1.5 kb (bottom).

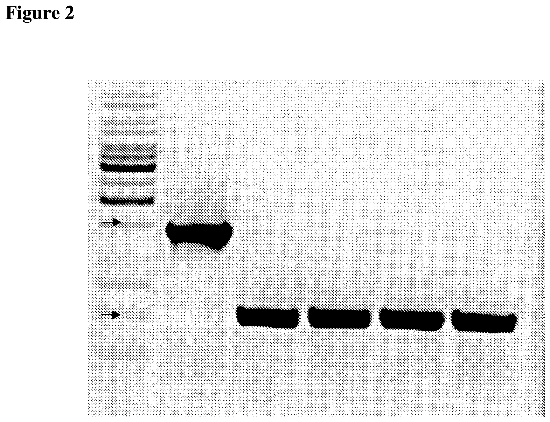

Figure 2 shows the PCR amplification products of *guaBA::cml* and *guaBA* deletions. Lane 1 is *guaBA*::cml; lanes 2 to 5 are *guaBA* deletions. Arrows indicate molecular weight marker bands of 1.5 kb (top) and 0.5 kb (bottom).

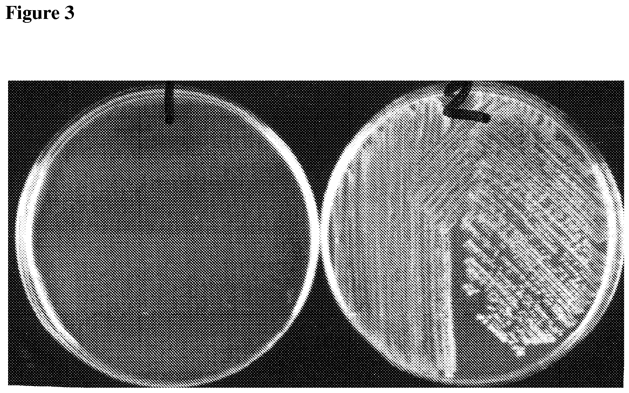

Figure 3 shows the results of a complementation study of the *guaBA* deletion. Plate 1 is the *guaBA* mutant transformed with pLowBlu 184; plate 2 is the same mutant transformed with pATGguaBA.

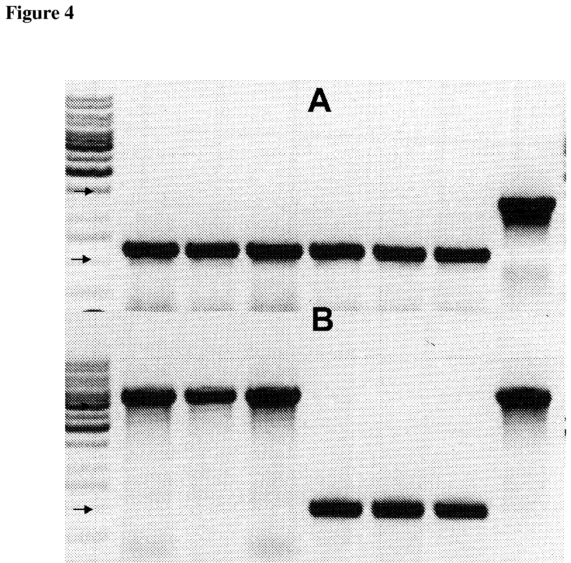

Figure 4 shows the PCR amplification products of wt, *clpX* and *clpX-guaBA* attenuated S. Paratyphi A. Panel A shows PCR products produced using primers specific for *clpX,* whereas panel B shows PCR products produced using primers specific for *guaBA.* Arrows indicate molecular weight marker bands of 1.5 kb (top) and 0.5 kb (bottom) on panel A, and 3 kb (top) and 0.5 kb (bottom) on panel B.

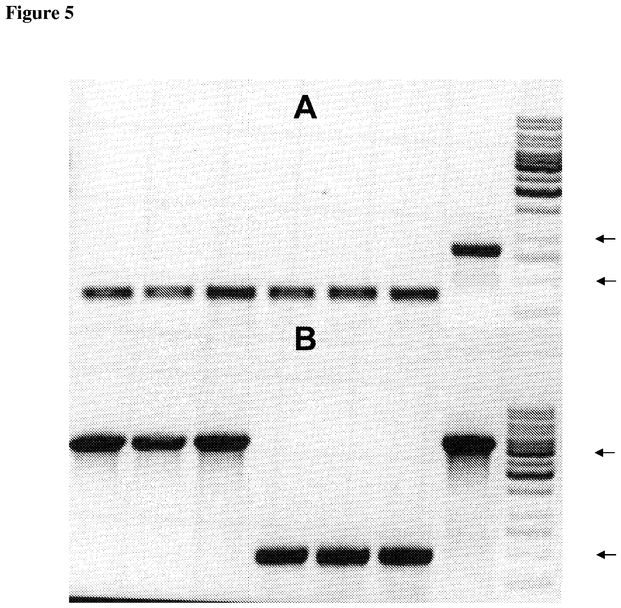

Figure 5 shows the PCR amplification products of wt, *clpP* and *clpP-guaBA* attenuated S. Paratyphi A. Panel A shows PCR products produced using primers specific for *clpP,* whereas panel B shows PCR products produced using primers specific for *guaBA.* Arrows indicate molecular weight marker bands of 1 kb (top) and 0.5 kb (bottom) on panel A, and 3 kb (top) and 0.5 kb (bottom) on panel B.

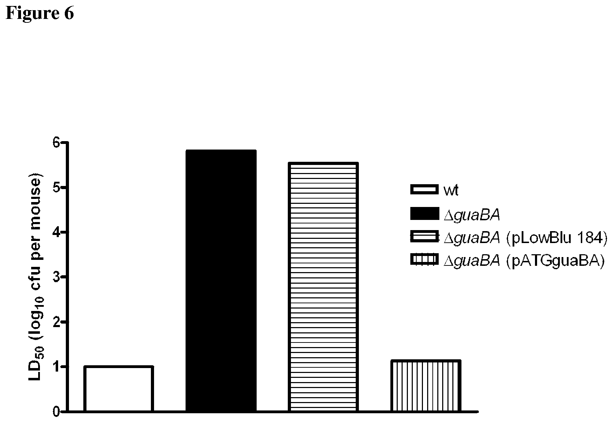

Figure 6 is a graphical representation of data from LD₅₀ tests in mice injected with wt, *guaBA-* deleted *S*. Paratyphi A, *guaBA*-deleted complemented with pLowBlu 184, and *guaBA*-deleted complemented with pATGguaBA.

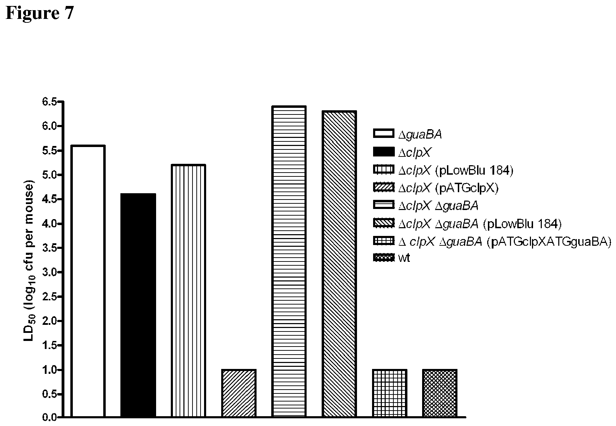

Figure 7 is a graphical representation of data from LD₅₀ tests in mice injected with wt, *clpX-* deleted S. Paratyphi A, *guaBA*-deleted *S.* Paratyphi A, or *clpX-guaBA-*deleted *S*. Paratyphi A. The data include mice injected with *clpx*-deleted *S.* Paratyphi A complimented with pLowBlu 184 or pATGclpX, as well as *clpX-guaBA*-deleted *S*. Paratyphi A complimented with pLowBlu 184 or pATGclpXATGguaBA.

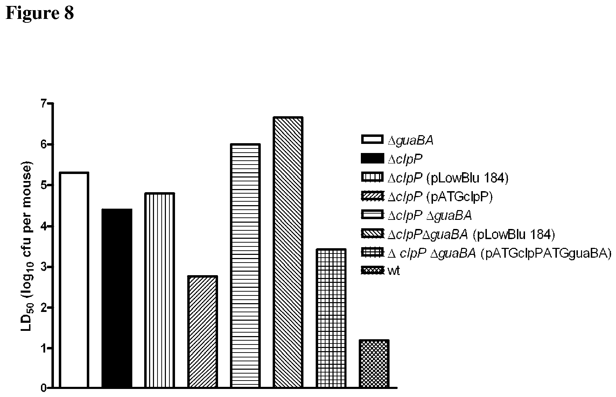

Figure 8 is a graphical representation of data from LD₅₀ tests in mice injected with wt, *clpP*-deleted *S.* Paratyphi A, *guaBA*-deleted *S.* Paratyphi A, or *clpP-guaBA*-deleted *S.* Paratyphi A. The data include mice injected with *clpP*-deleted S. Paratyphi A complimented with pLowBlu 184 or pATGclpP, as well as *clpP-guaBA*-deleted *S.* Paratyphi A complimented with pLowBlu 184 or pATGclpPATGguaBA.

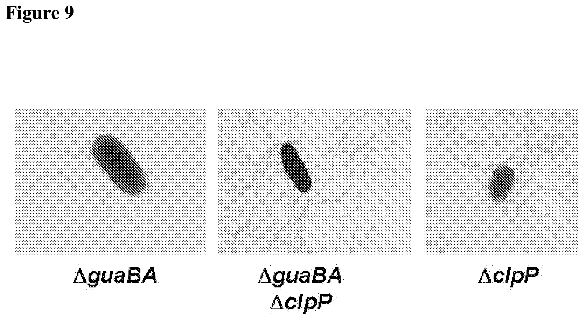

Figure 9 shows hyper-flagellation by electron microscopy of mutations clpP in S. Paratyphi A.

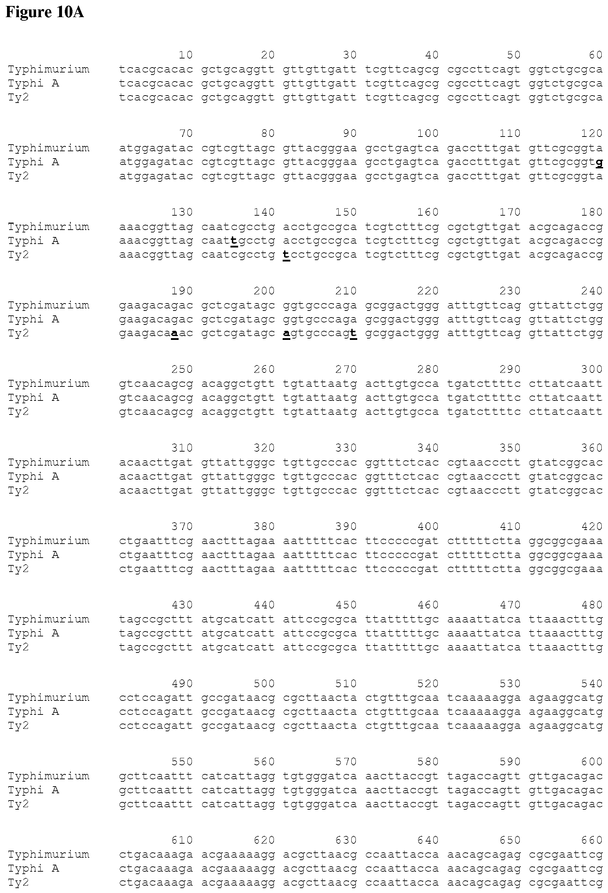

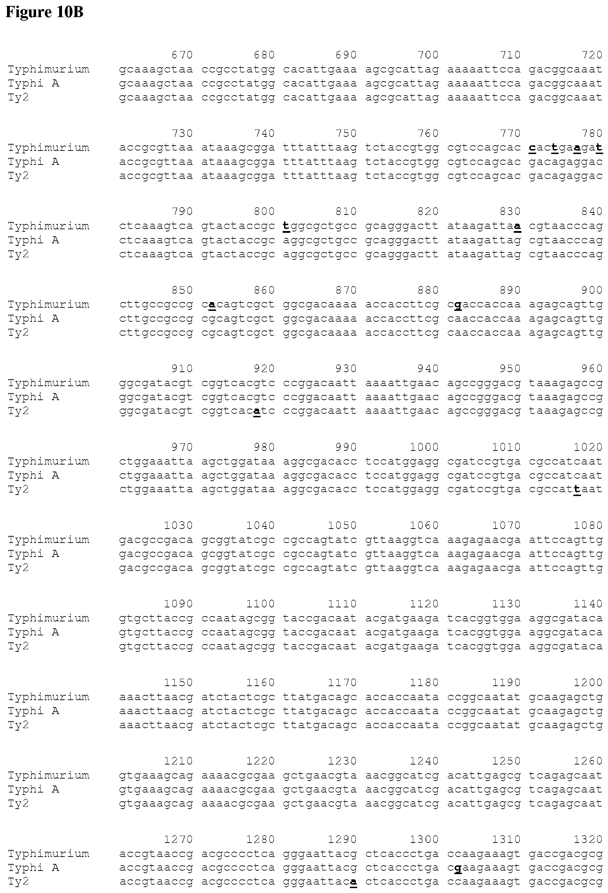

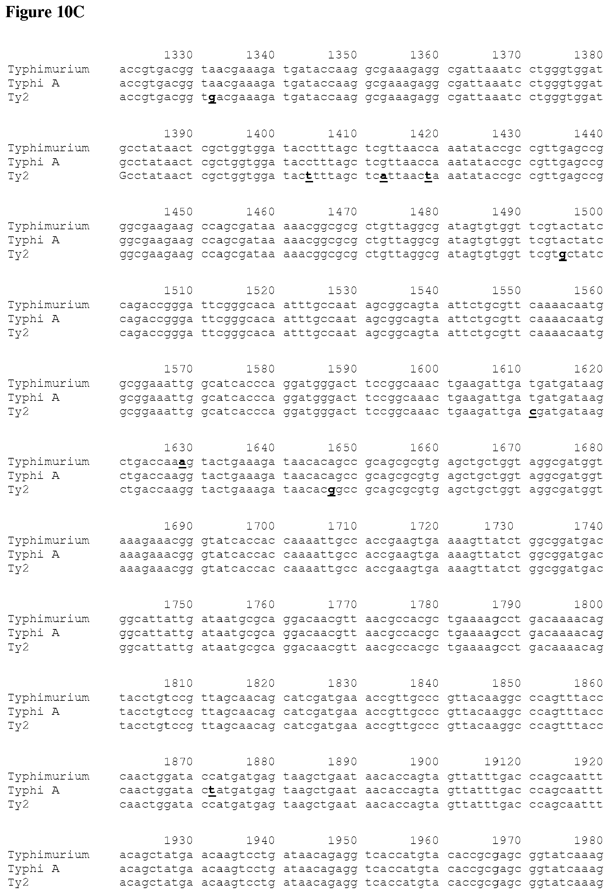

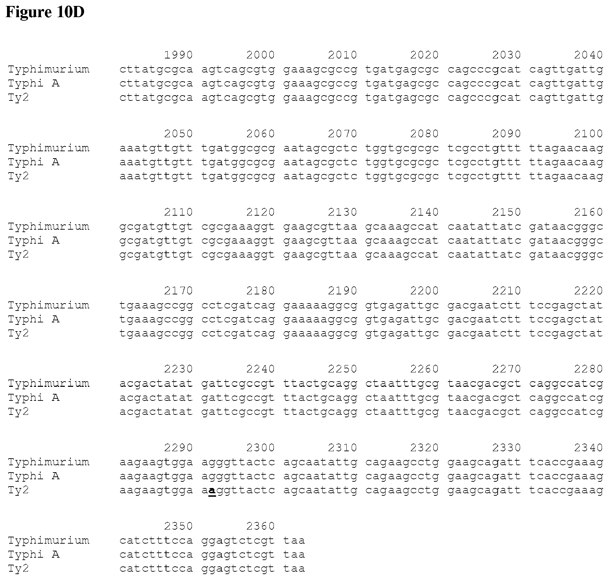

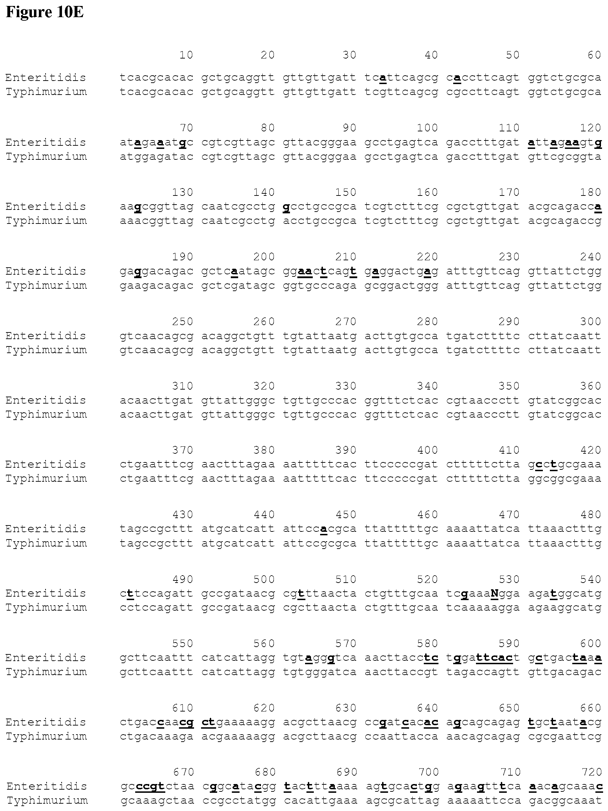

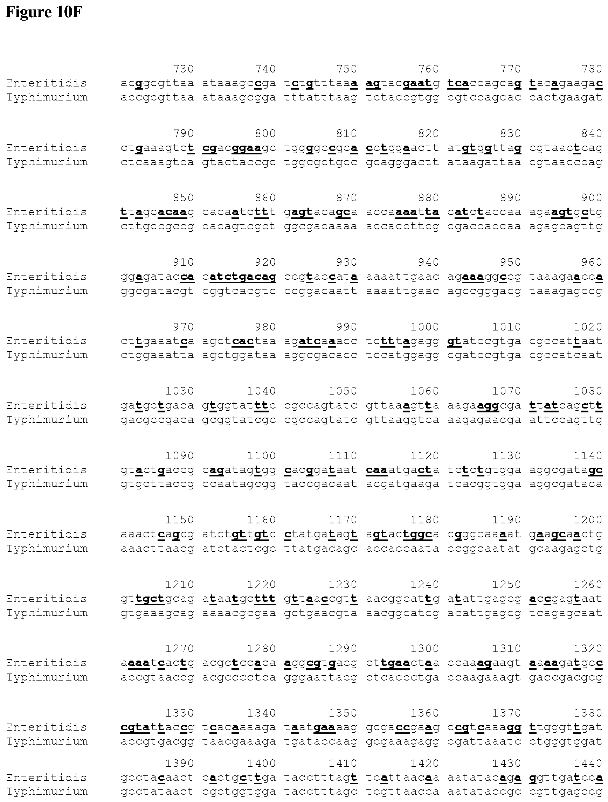

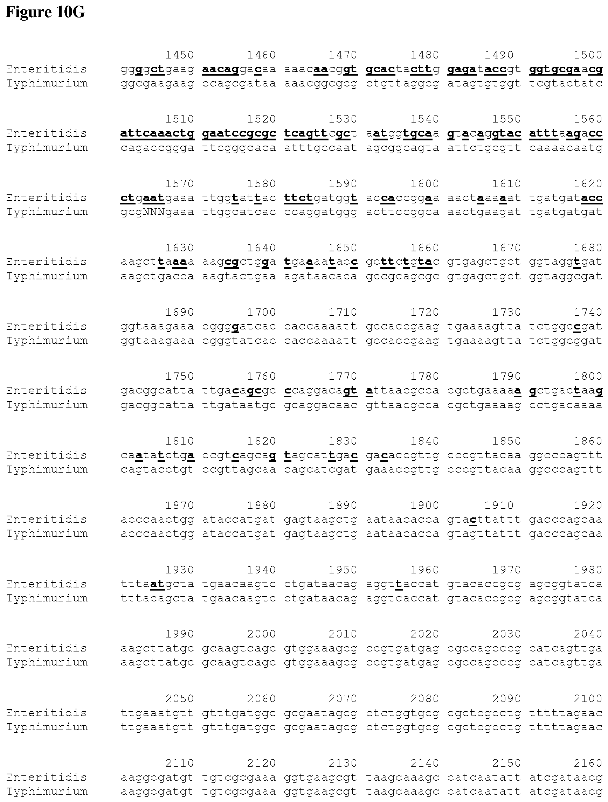

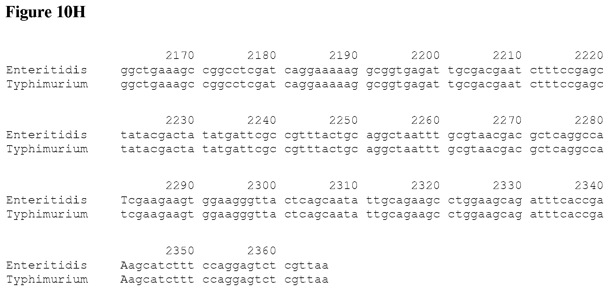

Figure 10 Alignment of chromosomal *fliD-fliS* loci from *Salmonella enterica* serovars Typhimurium, Enteritidis, Paratyphi A, and Typhi. Figures 10A-10D show the alignment of the chromosomal *fliD-fliS* loci as between *Salmonella* Typhimurium (SEQ ID NO:47), serovar Paratyphi A (using the shorthand "Typhi A") (SEQ ID NO:48) and serovar Typhi strain Ty2 (SEQ ID NO:49). Figures 10E-10H show the alignment of the chromosomal *fliD-fliS* loci as between *Salmonella* Enteritidis (SEQ ID NO:50) and *Salmonella* Typhimurium (SEQ ID NO:47).

### DETAILED DESCRIPTION OF THE INVENTION

### A. Attenuated S. Paratyphi A Strains

Disclosed herein is an attenuated S. Paratyphi A strain. Disclosed herein, such attenuated S. Paratyphi A strains may be used to induce an immune response in a subject without causing disease in the subject.

The S. Paratyphi A strain used as the starting material of the present invention may be any *S*. Paratyphi A strain and the identity of the strain is not critical. Preferred *S*. Paratyphi A strains include *S*. Paratyphi A 9150 strain.

The *S*. Paratyphi A strains for use in the present invention are attenuated. As used herein, attenuated strains of *S*. Paratyphi A are those that have a reduced, decreased, or suppressed ability to cause disease in a subject, or those completely lacking in the ability to cause disease in a subject. Attenuated strains may exhibit reduced or no expression of one or more genes, may express one or more proteins with reduced or no activity, may exhibit a reduced ability to grow and divide, or a combination of two or more of these characteristics. The attenuated strains of the present invention may be living or dead.

In addition to the attenuated *S*. Paratyphi A strains for use in the present invention, attenuated strains of other enteric pathogens (e.g., *Salmonella* Typhi, *Salmonella* Paratyphi B, *Shigella, Vibrio cholerae*), commensals (e.g., *Lactobacillus, Streptococcus gordonii*) and licensed vaccine strains (e.g., BCG) are also disclosed herein. These additional strains have all of the attenuating mutations of the *S*. Paratyphi A strains for use in the present invention, may be transformed with the stabilized plasmid expression system as disclosed herein, and may be used as an immunizing composition as described herein.

Disclosed herein, the attenuated *S*. Paratyphi A strains for use in the present invention have a mutation in one or more of the *guaBA* loci, the *guaB* gene, the *guaA* gene, the *clpP* gene and the *clpX* gene of S. Paratyphi. The attenuated *S*. Paratyphi A strains for use in the present invention may have a mutation (i) in the *guaB* gene and the *clpP* gene, (ii) in the *guaA* gene and the *clpP* gene, (iii) in the *guaB* gene, the *guaA* gene, and the *clpP* gene, (iv) in the *guaBA* loci and the *clpP* gene, (v) in the *guaB* gene, the *clpP* gene and the *clpX* gene, (vi) in the *guaA* gene, the *clpP* gene and the *clpX* gene*,* (vii) in the *guaB* gene, the *guaA* gene, the *clpP* gene and the *clpX* gene*,* or (xviii) in the *guaBA* loci, the *clpP* gene and the *clpX* gene*.*

The mutations of the loci and genes described herein may be any mutation, such as one or more nucleic acid deletions, insertions or substitutions. The mutations may be any deletion, insertion or substitution of the loci or genes that results in a reduction or absence of expression from the loci or genes, or a reduction or absence of activity of a polypeptide encoded by the loci or genes. The mutations may be in the coding or non-coding regions of the loci or genes.

Preferably, in the present invention, the chromosomal genome of the S. Paratyphi A strain is modified by removing or otherwise modifying the *guaBA* loci, and thus blocking the *de novo* biosynthesis of guanine nucleotides. More preferably, a mutation in the *guaBA* loci inactivates the purine metabolic pathway enzymes IMP dehydrogenase (encoded by *guaB*) and GMP synthetase (encoded by *guaA*)*.* As a consequence of these mutations, S. Paratyphi A are unable to *de novo* synthesize GMP, and consequently GDP and GTP nucleotides, which severely limits bacterial growth in mammalian tissues. *In vitro,* the Δ*guaBA S.* Paratyphi A mutants for use in the present invention are unable to grow in minimal medium unless supplemented with guanine. In tissue culture, the Δ*guaBA S.* Paratyphi A mutants for use in the present invention were found to show a significant reduction in their capability for invasion. Δ*guaBA S.* Paratyphi A mutants may scavenge guanine nucleotides from the tissues of the mammalian host. However, their assimilation into S. Paratyphi A requires prior dephosphorylation to nucleosides by periplasmic nucleotidases to be incorporated as nucleotide precursors into the guanine salvage pathway. Therefore, as nucleotides are readily available in the intracellular environment of the mammalian host, the attenuation due to the *de novo* synthesis of guanine nucleotides is due either to the inefficiency of the salvage pathway or to reasons that are obscure to today's knowledge.

The *guaA* gene of *S*. Paratyphi A 9150, which encodes GMP synthetase, is 1578 bp in size (SEQ ID NO:36), and is 98% homologous to the *guaA* gene of *S.* Typhi Ty2 as determined by NCBI BLAST nucleotide comparison. Deletion mutants can be produced by eliminating portions of the coding region of the *guaA* gene of *S*. Paratyphi A so that proper folding or activity of GuaA is prevented. For example, about 25 to about 1500 bp, about 75 to about 1400 bp, about 100 to about 1300 bp, or all of the coding region can be deleted. Alternatively, the deletion mutants can be produced by eliminating, for example, a 1 to 100 bp fragment of the *guaA* gene of *S*. Paratyphi A so that the proper reading frame of the gene is shifted. In the latter instance, a nonsense polypeptide may be produced or polypeptide synthesis may be aborted due to a frame-shift-induced stop codon. The preferred size of the deletion is about 75 to 750 bp. Deletions can also be made that extend beyond the *guaA* gene, i.e., deletions in the elements controlling translation of the *guaA* gene, such as in a ribosome binding site.

The *guaB* gene of S. Paratyphi A 9150, which encodes IMP dehydrogenase, is 1467 bp in size (SEQ ID NO:35), and is 98% homologous to the *guaB* gene of *S*. Typhi Ty2 as determined by NCBI BLAST nucleotide comparison. Deletion mutants can be produced by eliminating portions of the coding region of the *guaB* gene of *S*. Paratyphi A so that proper folding or activity of GuaB is prevented. For example, about 25 to about 1400 bp, about 75 to about 1300 bp, about 100 to about 1200 bp, or all of the coding region can be deleted. Alternatively, the deletion mutants can be produced by eliminating, for example, a 1 to 100 bp fragment of the *guaB* gene of S. Paratyphi A so that the proper reading frame of the gene is shifted. In the latter instance, a nonsense polypeptide may be produced or polypeptide synthesis may be aborted due to a frame-shift-induced stop codon. The preferred size of the deletion is about 75 to 750 bp. Deletions can also be made that extend beyond the *guaB* gene, i.e., deletions in the elements controlling transcription of the *guaB* gene, such as in a promoter.

The *clpP* gene of S. Paratyphi A 9150, which encodes a serine-protease, is 624 bp in size (SEQ ID NO:37), and 99% homologous to the *clpP* gene of S. Typhi Ty2 as determined by NCBI BLAST nucleotide comparison. Deletion mutants can be produced by eliminating portions of the coding region of the *clpP* gene of *S*. Paratyphi A so that proper folding or activity of ClpP is prevented. For example, about 25 to about 600 bp, about 75 to about 500 bp, about 100 to about 400 bp, or all of the coding region can be deleted. Alternatively, the deletion mutants can be produced by eliminating, for example, a 1 to 100 bp fragment of the *clpP* gene of *S*. Paratyphi A so that the proper reading frame of the gene is shifted. In the latter instance, a nonsense polypeptide may be produced or polypeptide synthesis may be aborted due to a frame-shift-induced stop codon. The preferred size of the deletion is about 25 to 600 bp. Deletions can also be made that extend beyond the *clpP* gene, i.e., deletions in the elements controlling transcription of the *clpP* gene, such as in a promoter.

The *clpX* gene of *S*. Paratyphi A 9150, which encodes a chaperone ATPase, is 1272 bp in size (SEQ ID NO:38), and 99% homologous to the *clpX* gene of *S*. Typhi Ty2 as determined by NCBI BLAST nucleotide comparison. Deletion mutants can be produced by eliminating portions of the coding region of the *clpX* gene of *S*. Paratyphi A so that proper folding or activity of ClpX is prevented. For example, about 25 to about 1200 bp, about 75 to about 1100 bp, about 100 to about 1000 bp, or all of the coding region can be deleted. Alternatively, the deletion mutants can be produced by eliminating, for example, a 1 to 100 bp fragment of the *clpX* gene of *S*. Paratyphi A so that the proper reading frame of the gene is shifted. In the latter instance, a nonsense polypeptide may be produced or polypeptide synthesis may be aborted due to a frame-shift-induced stop codon. The preferred size of the deletion is about 75 to 750 bp. Deletions can also be made that extend beyond the *clpX* gene, i.e., deletions in the elements controlling transcription of the *clpX* gene, such as in a promoter.

Deletions can be made in any of the loci or genes included herein by using convenient restriction sites located within the loci or genes, or by site-directed mutagenesis with oligonucleotides (Sambrook et al, In: Molecular Cloning, A Laboratory Manual, Eds., Cold Spring Harbor Publications (1989)).

Inactivation of the loci or genes can also be carried out by an insertion of foreign DNA using any convenient restriction site, or by site-directed mutagenesis with oligonucleotides (Sambrook et al, supra) so as to interrupt the correct transcription of the loci or genes. The typical size of an insertion that can inactivate the loci or genes is from 1 base pair to 100 kbp, although insertions smaller than 100 kbp are preferable. The insertion can be made anywhere inside the loci or gene coding regions or between the coding regions and the promoters.

Other methods for the inactivation of the loci and genes include the transfer into *Salmonella* of deletions or insertions made in other enterobacteriae *guaBA* loci, *guaA, guaB, clpP* or *clpX* genes, transposon-generated deletions, and imprecise excision of DNA insertions.

Preferably, the bacterial loci and genes are mutated using Lambda Red-mediated mutagenesis (Datsenko and Wanner, PNAS USA 97:6640-6645 (2000)). Briefly, in step 1 host bacteria targeted for mutation are transformed with a temperature sensitive plasmid encoding λ Red recombinase. These bacteria are grown in the presence of arabinose to induce λ Red production. Chromosomal mutagenesis of a target sequence is accomplished by electroporation of the host with linear DNA in which the target gene is replaced with an antibiotic resistance marker. This DNA also encodes short regions of flanking chromosomal sequences to allow for chromosomal integration of the resistance marker by λ Red-mediated homologous recombination. Recombinants are selected for on solid media containing the appropriate antibiotic, and incubated at a temperature facilitating the loss of the plasmid encoding λ Red recombinase. For step 2, removal of the chromosomal resistance marker is facilitated by transforming the bacteria with a temperature sensitive plasmid encoding FLP recombinase, which targets unique sequences within the antibiotic resistance marker now present in the host chromosome. Transformants are grown at temperatures permissive for the presence of the FLP recombinase which is expressed constitutively. Mutants are screened via PCR, grown at a temperature to facilitate loss of the plasmid encoding FLP recombinase, and selected for storage.

The attenuated *S*. Paratyphi A strains for use in the present invention may contain mutations in one or more additional genes. While an extensive discussion of additional attenuating mutations of *Salmonella* spp. is provide in U.S. Patent No. 6,682,729, exemplary genes include those encoding various biochemical pathways, global regulatory systems, heat shock proteins, other regulatory genes, and putative virulence properties. Specific examples of such attenuating mutations include, but are not limited to: (i) auxotrophic mutations, such as *aro,* gua, *nad, thy,* and *asd* mutations; (ii) mutations that inactivate global regulatory functions, such as *cya, crp, phoP*/*phoQ, phoP^{c}* and *ompR* mutations; (iii) mutations that modify the stress response, such as *recA, htrA, htpR, hsp* and *groEL* mutations; (iv) mutations in specific virulence factors, such as *pag* and *prg* (v) mutations that affect DNA topology, such as *topA* mutations; (vi) mutations that block biogenesis of surface polysaccharides, such as *rfb, galE* and *via* mutations; (vii) mutations that modify suicide systems, such as *sacB, nuc, hok, gef, kil,* and *phlA* mutations; (viii) mutations that introduce suicide systems, such as lysogens encoded by *P22,* λ murein transglycosylase and S-gene; and (ix) mutations that disrupt or modify the correct cell cycle, such as *minB* mutations.

### B. Stabilized Expression Plasmid System

The attenuated S. Paratyphi A strains for use in the present invention include those strains engineered to express selected polypeptides (antigens). Such attenuated S. Paratyphi A strains can be used to induce an immune response to *S*. Paratyphi itself, or to induce an immune response to the selected antigens expressed by the attenuated *S*. Paratyphi A strains, or both.

Such attenuated *S*. Paratyphi A strains are transformed with a stabilized expression plasmid system. The stabilized expression plasmid system encodes a selected antigen.

The stabilized expression plasmid system comprises expression vector that comprises a plasmid maintenance system (PMS) and a nucleotide sequence encoding a selected antigen.

The stabilized expression plasmid system optimizes the maintenance of the expression vector in the bacteria at two independent levels by: (1) removing sole dependence on balanced lethal maintenance systems; and (2) incorporating a plasmid partition system to prevent random segregation of expression vectors, thereby enhancing their inheritance and stability.

The PMS includes (a) an origin of replication, (b) at least one post-segregational killing function, and (c) at least one partitioning function. Each of the noted elements of the PMS may be an individual cassette of the stabilized expression plasmid system. Each of the cassettes may comprise unique restriction enzyme cleavage sites located at the 5' and 3' ends of the cassettes.

Preferred stabilized expression plasmid systems are those described in pending U.S. patent application publication number US 2007/0281348.

### 1. Origin of Replication

The PMS includes a restricted-copy-number origin of replication that limits the expression vector to a range of plasmid copies per cell. Due to varying degrees of toxicity associated with different selected antigens (e.g., higher toxicity for antigens derived from parasitic organisms such *Plasmodium falciparum* versus virtually no toxicity for the fragment C of tetanus toxin), the stabilized expression plasmid system of the present invention is based on either a low or medium copy number expression vector (plasmid). It will be appreciated by one skilled in the art that the selection of an origin of replication will depend on the degree of toxicity, i.e., the copy number should go down as toxicity to the bacterial strain goes up.

It is preferable for the origin of replication to confer an average copy number which is between about 2 and about 75 copies per cell, between about 5 and about 60 copies per cell, between about 5 to about 30 copies per cell, or between about 5 to about 15 copies per cell. The origins of replication included herein are derived from the *E*. *coli* plasmid pAT153 (*ori*E1*,* ∼60 copies per chromosomal equivalent), the *E. coli* plasmid pACYC184 (*ori*15A*,* ∼15 copies per chromosomal equivalent), and the *Salmonella typhimurium* plasmid pSC101 (*ori*101*,* ∼5 copies per chromosomal equivalent). The structural organization of the engineered origins of replication cassettes for pSC101, pACYC184, and pAT153 are analogous in structure and function.

The origins of replication for use in the present invention includes both naturally-occurring origins of replication, as well as origins of replication encoded by nucleotide sequences which are substantially homologous to nucleotide sequences encoding naturally-occurring origins of replication, and which retain the function exhibited by the naturally-occurring origins of replication.

In preferred embodiments, the nucleotide sequence encoding the origin of replication is a nucleotide sequence selected from the group consisting of the *ori*E1 sequence of SEQ ID NO:28, the *ori*101 sequence of SEQ ID NO:30, and the *ori*15A sequence of SEQ ID NO:29.

In a further preferred embodiment, the origin of replication is the *ori*E1 locus from pSC101, conferring a copy number of approximately 5 copies per genome equivalent, set forth in SEQ ID NO:28.

### 2. Partitioning Function

The PMS also includes a partitioning function, also known in the art and herein as a "segregating system" and a "partitioning system." The partitioning function is any plasmid stability-enhancing function that operates to increase the frequency of successful delivery of a plasmid to each newly divided bacterial cell, as compared to the frequency of delivery of a corresponding plasmid without such a function. Partitioning systems include, for example, equi-partitioning systems, pair-site partitioning systems, and the systems provided in Table 1 of Chapter 5, Partition Systems of Bacterial Plasmids. B.E. Funnell and R.A. Slavcev. In Plasmid Biology. 2004. BE Funnell and GJ Phillips, eds. ASM Press, Washington, DC.

The partitioning systems for use in the present invention includes both naturally-occurring partitioning systems, as well as partitioning systems encoded by nucleotide sequences which are substantially homologous to nucleotide sequences encoding naturally-occurring partitioning systems, and which retain the function exhibited by the naturally-occurring partitioning systems.

Exemplary partitioning functions include, without limitation, systems of pSC101, the F factor, the P1 prophage, and IncFII drug resistance plasmids.

In particular, the *par* passive partitioning locus can be used. The function of the *par* locus appears to be related to increasing plasmid supercoiling at the origin of replication, which is also the binding site for DNA gyrase. An exemplary *par* sequence is that of *E. coli,* set forth in SEQ ID NO:32 (Miller et al. Nucleotide sequence of the partition locus of Escherichia coli plasmid pSC101, Gene 24:309-15 (1983); GenBank accession no. X01654, nucleotides 4524 - 4890)).

The active partitioning *parA* locus may also be used. An exemplary *parA* locus sequence is set forth in SEQ ID NO:31.

### 3. Post-Segregational Killing Function

The PMS further includes at least one post-segregational killing (PSK) function. The PSK function is a function which results in the death of any newly divided bacterial cell which does not inherit the plasmid of interest, and specifically includes balanced-lethal systems such as *asd* or *ssb,* proteic systems such as *phd-doc,* and antisense systems such as *hok-sok.*

The PSK function for use in the present invention includes both naturally-occurring PSK functions, as well as PSK functions encoded by nucleotide sequences which are substantially homologous to nucleotide sequences encoding naturally-occurring PSK functions, and which retain the function exhibited by the naturally-occurring PSK functions.

In preferred embodiments, the PSK function is the *ssb* balanced lethal system. The single-stranded binding protein (SSB) from S. Typhi is used to trans-complement an otherwise lethal mutation introduced into the chromosomal *ssb* gene. The biochemistry and metabolic roles of the *E. coli* SSB protein have been extensively reviewed in Lohman et al., Annual Reviews in Biochemistry 63:527, 1994 and Chase et al., Annual Reviews in Biochemistry 55:103, 1986.

In the S. Paratyphi A strains for use in the present invention comprising a stabilized expression plasmid system wherein the PSK function is the *ssb* balanced lethal system, the native *ssb* locus of the bacteria is inactivated. The native *ssb* locus may be inactivated by any means known in the art, such as a suicide vector comprising a temperature sensitive origin of replication or Lambda Red-mediated mutagenesis (Datsenko and Wanner, PNAS USA 97:6640-6645 (2000)). In a preferred aspect, Lambda Red-mediated mutagenesis is used to inactivate the *ssb* locus of the attenuated S. Paratyphi A strains of the present invention.

In another aspect of the invention, the PSK function is the *ssb* locus where both the inducible and the constitutive *ssb* gene promoters are used as the promoters of the *ssb* PSK function. In a preferred embodiment, the PSK function comprises a *ssb* inducible promoter, a *ssb* constitutive promoter and a *ssb* coding region. Preferably, the *ssb* locus is the *ssb* locus of any one of *Shigella flexneri,* Salmonella Typhi and *E. coli.* In one embodiment the *ssb* locus is the *ssb* locus of *S*. *flexneri* 2a strain CVD 1208s set forth in SEQ ID NO:34.

In a related aspect of the invention, mutated alleles such as *ssb*-1 (or any mutation functionally equivalent to this allele, such as W54S; Carlini et al. Mol. Microbiol. 10:1067-1075 (1993)) may be incorporated into the stabilized expression plasmid system to enhance higher copy number plasmids by over-expression of SSB1-like proteins to form the required biologically active tetramers of SSB.

In a further embodiment, the PMS comprises two PSK functions.

### 4. Selected Antigen

The stabilized expression plasmid system also comprises a polynucleotide encoding selected antigen under control of a promoter.

The promoter is preferably an environmentally regulatable promoter, controlled by a biologically relevant signal such as osmolarity. In a preferred embodiment, the promoter is the *ompC* promoter. The *ompC* gene encodes a porin protein which inserts as a trimer into the outer membrane of a bacterial cell. Expression and control of *ompC* has been reviewed in considerable detail in Pratt et al., Molecular Microbiology 20:911, 1996 and Egger et al., Genes to Cells 2:167, 1997. In a preferred embodiment the *ompC* promoter fragment from E. *coli* is set forth in SEQ ID NO:33. See U.S. Patent No.: 6,703,233. Transcription of this cassette may be terminated in the 3'-distal region by a *trpA* transcriptional terminator.

In one aspect, the inducible promoter is the mutated P_{*ompC*1}*,* or the P_{*ompC*3} promoter. The promoter may be used to exclusively control the transcription of the downstream selected antigen.

The invention encompasses the expression of any antigen which does not destroy the attenuated S. Paratyphi A strain expressing it, and which elicits an immune response when the attenuated S. Paratyphi A strain expressing the antigen is administered to the subject. The selected antigens may be homologous (from S. Paratyphi A) or heterologous.

Non-limiting examples of the selected antigen include: Shiga toxin 1 (Stx1) antigen, Shiga toxin 2 (Stx2) antigen, hepatitis B, *Haemophilus influenzae* type b, hepatitis A, acellular pertussis (_{ac}P), varicella, rotavirus, *Streptococcus pneumoniae* (pneumococcal), and *Neisseria meningitidis* (meningococcal). See Ellis et al., Advances in Pharm., 39: 393423, 1997. Where the antigen is a Shiga toxin 2 antigen, the Shiga toxin 2 antigen can, for example, be either a B subunit pentamer or a genetically detoxified Stx 2. Further antigens of relevance to biodefense include: 1) one or more domains of the anthrax toxin Protective Antigen PA83 moiety, including but not limited to domain 4 (the eukaryotic cell-binding domain; D4), the processed 63 kDa biologically active form of PA83, or full-length PA83; and 2) *Clostridium botulinum* antigens comprising the eukaryotic cell-binding heavy chain fragment of any neurotoxin serotype A, B, C, D, E, F, or G, in any combination. Other selected antigens include each of those disclosed in U.S. Patent No. 6,190,669.

In one aspect, the selected antigen is an antigen that induced an immune response to cancer. In another aspect, the selected antigen is designed to provoke an immune response to autoantigens, B cell receptors and/or T cell receptors which are implicated in autoimmune or immunological diseases. For example, where inappropriate immune responses are raised against body tissues or environmental antigens, the immunizing compositions of the present invention may be used to induce an immune response to the autoantigens, B cell receptors and/or T cell receptors to modulate the responses and ameliorate the diseases. For example, such techniques can be efficacious in treating myasthenia gravis, lupus erythematosis, rheumatoid arthritis, multiple sclerosis, allergies and asthma.

In another aspect of the present invention, the stabilized expression plasmid system may include a polynucleotide encoding a selectable marker, or a temperature sensitive marker, such as drug resistance marker. A non-limiting example of a drug resistance marker includes *aph* which is known in the art to confer resistance to aminoglycosides kanamycin and/or neomycin.

The term "substantially homologous" or "substantial homologue," in reference to a nucleotide sequence or amino acid sequence herein, indicates that the nucleic acid sequence or amino acid sequence has sufficient homology as compared to a reference sequence (e.g., a native or naturally-occurring sequence) to permit the sequence to perform the same basic function as the corresponding reference sequence; a substantially homologous sequence is typically at least about 70 percent sequentially identical as compared to the reference sequence, typically at least about 85 percent sequentially identical, preferably at least about 90 or 95 percent sequentially identical, and most preferably about 96, 97, 98 or 99 percent sequentially identical, as compared to the reference sequence. It will be appreciated that throughout the specification, where reference is made to specific nucleotide sequences and/or amino acid sequences, that such nucleotide sequences and/or amino acid sequences may be replaced by substantially homologous sequences.

### C. Methods of Inducing an Immune Response

Disclosed herein are methods of inducing an immune response in a subject. The immune response may be to the attenuated S. Paratyphi A strain itself, a selected antigen expressed by an attenuated S. Paratyphi A strain transformed with a stabilized expression plasmid system, or both.

Disclosed herein, the method of inducing an immune response comprises administering one or more of the strains disclosed herein to a subject in an amount sufficient to induce an immune response in the subject. As used herein, the strain includes both untransformed and transformed attenuated S. Paratyphi A strains.

Disclosed herein, the method of inducing an immune response comprises administering a pharmaceutical formulation comprising one or more of the strains as disclosed herein to a subject in an amount sufficient to induce an immune response in the subject (an immunologically-effective amount).

For the sake of convenience, the strains as disclosed herein and pharmaceutical formulations comprising the strains are referred to herein as "immunizing compositions." The skilled artisan will appreciate that the immunizing compositions are synonymous with vaccines.

As used herein, an "immune response" is the physiological response of the subject's immune system to the immunizing composition. An immune response may include an innate immune response, an adaptive immune response, or both.

Disclosed herein, the immune response is a protective immune response. A protective immune response confers immunological cellular memory upon the subject, with the effect that a secondary exposure to the same or a similar antigen is characterized by one or more of the following characteristics: shorter lag phase than the lag phase resulting from exposure to the selected antigen in the absence of prior exposure to the immunizing composition; production of antibody which continues for a longer period than production of antibody resulting from exposure to the selected antigen in the absence of prior exposure to the immunizing composition; a change in the type and quality of antibody produced in comparison to the type and quality of antibody produced upon exposure to the selected antigen in the absence of prior exposure to the immunizing composition; a shift in class response, with IgG antibodies appearing in higher concentrations and with greater persistence than IgM, than occurs in response to exposure to the selected antigen in the absence of prior exposure to the immunizing composition; an increased average affinity (binding constant) of the antibodies for the antigen in comparison with the average affinity of antibodies for the antigen resulting from exposure to the selected antigen in the absence of prior exposure to the immunizing composition; and/or other characteristics known in the art to characterize a secondary immune response.

The subject to which the immunizing compositions may be administered is preferably a human, but may also be another mammal such as a simian, dog, cat, horse, cow or pig, or a bird, such as a chicken.

Disclosed herein, the subject is a subject at risk for developing an *S*. Paratyphi A infection. Disclosed herein, the subject is immunologically naive or, alternatively, exhibits pre-existing immunity to *S*. Typhi infection or *S*. Paratyphi A infection.

Disclosed herein, the subject to which the strains of the present invention are administered develops a protective immune response against paratyphoid fever.

### D. Formulations, Dosages, and Modes of Administration

The attenuated strains disclosed herein, both those untransformed and transformed with a stabilized expression plasmid system, may be administered to a subject to induce an immune response. The strains disclosed herein are administered in a pharmaceutical formulation.

The pharmaceutical formulations disclosed herein may include pharmaceutically acceptable carriers, excipients, other ingredients, such as adjuvants. Pharmaceutically acceptable carriers, excipients, other ingredients are those compounds, solutions, substances or materials that are compatible with the strains and are not unduly deleterious to the recipient thereof. In particular, carriers, excipients, other ingredients are those useful in preparing a pharmaceutical formulation that is generally safe, non-toxic and neither biologically nor otherwise undesirable, and that may present pharmacologically favorable profiles, and includes carriers, excipients, other ingredients that are acceptable for veterinary use as well as human pharmaceutical use.

Suitable pharmaceutically acceptable carriers and excipients are well known in art and can be determined by those of skill in the art as the clinical situation warrants. The skilled artisan will understand that diluents are included within the scope of the terms carriers and excipients. Examples of suitable carriers and excipients include saline, buffered saline, dextrose, water, glycerol, ethanol, more particularly: (1) Dulbecco's phosphate buffered saline, pH about 7.4, containing about 1 mg/ml to 25 mg/ml human serum albumin, (2) 0.9% saline (0.9% w/v NaCl), (3) 5% (w/v) dextrose, and (4) water.

The mode of administration of the immunizing compositions disclosed herein may be any suitable delivery means and/or methods that results in the induction of an immune response in the subject. Delivery means may include, without limitation, parenteral administration methods, such as subcutaneous (SC) injection, intravenous (IV) injection, transdermal, intramuscular (IM), intradermal (ID), as well as non-parenteral, e.g., oral, nasal, intravaginal, pulmonary (inhalation), ophthalmic, rectal administration, or by any other mode that results in the immunogenic composition contacting mucosal tissues. Disclosed herein, administration is orally.

Disclosed herein, the immunizing compositions exists as an atomized dispersion for delivery by inhalation. Various liquid and powder formulations can be prepared by conventional methods for inhalation into the lungs of the subject to be treated. The atomized dispersion of the immunizing compositions typically contains carriers common for atomized or aerosolized dispersions, such as buffered saline and/or other compounds well known to those of skill in the art. The delivery of the immunogenic compositions via inhalation has the effect of rapidly dispersing the immunizing compositions to a large area of mucosal tissues as well as quick absorption by the blood for circulation of the immunizing compositions.

Additionally, immunizing compositions disclosed herein also exist in a liquid form. The liquid can be for oral dosage, for ophthalmic or nasal dosage as drops, or for use as an enema or douche. When the immunizing composition is formulated as a liquid, the liquid can be either a solution or a suspension of the immunizing composition. There are a variety of suitable formulations for the solution or suspension of the immunizing composition that are well known to those of skill in the art, depending on the intended use thereof. Liquid formulations for oral administration prepared in water or other aqueous vehicles may contain various suspending agents such as methylcellulose, alginates, tragacanth, pectin, kelgin, carrageenan, acacia, polyvinylpyrrolidone, and polyvinyl alcohol. The liquid formulations may also include solutions, emulsions, syrups and elixirs containing, together with the immunizing compositions, wetting agents, sweeteners, and coloring and flavoring agents.

Delivery of the described immunizing compositions in liquid form via oral dosage exposes the mucosa of the gastrointestinal and urogenital tracts to the immunizing compositions. A suitable dose, stabilized to resist the pH extremes of the stomach, delivers the immunizing composition to all parts of the gastrointestinal tract, especially the upper portions thereof. Any methods of stabilizing the immunizing composition in a liquid oral dosage such that the effective delivery of the composition is distributed along the gastrointestinal tract are contemplated for use with the immunizing compositions described herein, including capsules and a resuspended buffer solution to protect the attenuated bacteria against the acidic pH. The particular pharmaceutically acceptable carriers or diluents employed are not critical to the present invention, and are conventional in the art. Examples of diluents include: buffers for buffering against gastric acid in the stomach, such as citrate buffer (pH 7.0) containing sucrose, bicarbonate buffer (pH 7.0) alone or bicarbonate buffer (pH 7.0) containing ascorbic acid, lactose, and optionally aspartame (Levine et al, Lancet, II:467-470 (1988)). Examples of carriers include: proteins, e.g., as found in skim milk; sugars, e.g., sucrose; or polyvinylpyrrolidone.

Delivery of the described immunizing compositions in liquid form via ophthalmic drops exposes the mucosa of the eyes and associated tissues to the immunizing compositions. A typical liquid carrier for eye drops is buffered and contains other compounds well known and easily identifiable to those of skill in the art.

Delivery of the described immunizing compositions in liquid form via nasal drops or aerosol exposes the mucosa of the nose and sinuses and associated tissues to the immunizing compositions. Liquid carriers for nasal drops are typically various forms of buffered saline.

Injectable formulations of the immunizing compositions may contain various carriers such as vegetable oils, dimethylacetamide, dimethylformamide, ethyl lactate, ethyl carbonate, isopropyl myristate, ethanol, polyols (glycerol, propylene glycol, and liquid polyethylene glycol) and the like. Physiologically acceptable excipients may include, for example, 5% dextrose, 0.9% saline, Ringer's solution or other suitable excipients. Intramuscular preparations can be dissolved and administered in a pharmaceutical excipient such as Water-for-Injection, 0.9% saline, or 5% glucose solution.

Disclosed herein, the attenuated S. Paratyphi A strains may be administered to a subject in conjunction with other suitable pharmacologically or physiologically active agents, e.g., antigenic and/or other biologically active substances.

Disclosed herein, the attenuated S. Paratyphi A strains comprising a stabilized expression plasmid system may be administered to a subject prior to, concurrent with, or after expression of the selected antigen has begun. Disclosed herein, the attenuated S. Paratyphi A strain comprising a stabilized expression plasmid system may be cultured for a period of time prior to administration to a subject to enable the bacterial to produce sufficient amounts of the selected antigen, such that an immune response will be raised to the selected antigen upon administration of the bacteria.

Disclosed herein, the amount and rate of administration of the immunizing compositions may be readily determined by those of ordinary skill in the art without undue experimentation, such as by use of conventional antibody titer determination techniques and conventional bioefficacy/biocompatibility protocols. The amount and rate of administration will vary based on factors such as the weight and health of the subject, the identity of the bacteria being administered to the subject, the identity of the polypeptide being expressed in those stains engineered to express a selected antigen, the desired therapeutic effect, the desired time span of bioactivity, and the mode of administration of the immunizing composition.

In general, the amount of an immunizing composition administered to a subject is an amount sufficient to induce an immune response in the subject to a *S*. Paratyphi A strain or to the selected antigen being expressed by the *S*. Paratyphi A strain (an immunologically-effective amount). Disclosed herein, the immune response is a protective immune response.

Disclosed herein, the dosage employed will contain about 10² cfu to 10¹⁰ cfu of the *S*. Paratyphi A strain, preferably about 10² cfu to 10⁷ cfu, or about 10⁶ cfu to 10⁹ cfu. Disclosed herein, formulations for oral administration comprise about 10² cfu to 10¹⁰ cfu of the *S*. Paratyphi A strain, preferably about 10⁶ cfu to 10⁹ cfu, and the formulation is in a capsule or resuspended in a buffer solution to protect the attenuated bacteria against the acidic pH in the stomach. Disclosed herein, formulations for nasal administration comprise about 10² cfu to 10¹⁰ cfu of the *S*. Paratyphi A strain, preferably about 10² cfu to 10⁷ cfu, and is used for intranasal administration in which the bacteria is given in drops or in aerosol.

Disclosed herein, the immunizing compositions may be administered in a single dose, or in multiple doses over prolonged periods of time. Disclosed herein, the immunizing compositions may be administered over a period of one week, two weeks, three weeks, one month, six weeks, two months, ten weeks, three months, four months, six months, one year, or for extended periods longer than one year.

Disclosed herein, the immunizing compositions may be provided in dosage unit for uniform dosage and ease of administration. Each dosage unit form contains a predetermined quantity of the strains of the present invention calculated to produce a desired immune response, in association with a pharmaceutically acceptable carrier, excipient, or other ingredient.

Disclosed herein is a kit comprising one or more of the immunizing compositions, and optionally means for administering the compositions, and instructions for administering the compositions.

### E. EXAMPLES

### 1. Bacterial strains and culturing conditions

*Escherichia coli* strain DH5 alpha was used for all plasmid constructions. Live attenuated *S*. Typhi strain CVD 908*-htrA* harbors deletion mutations in *aroC* and *aroD,* interrupting the aromatic compound biosynthesis pathway, and *htrA,* which encodes a stress response protein (see Infect Immun. 60:2 (1992), pp. 536-541 and J. Biotechnol. 44:1-3 (1996), pp. 193-196). *S*. Paratyphi A 9150 lot # 11848 was purchased from the American Type Culture Collection (Manassas, VA), and stored as CV 223 and CV 224. CV 223 was used in all experiments.

*E. coli* DH5 alpha was grown using Luria Bertani (LB) liquid medium or agar (Difco, Detroit, Mich) supplemented with antibiotics carbenicillin (carb; 50 µg/ml), kanamycin (kan; 50 µg/ml) or chloramphenicol (cml; 25 µg/ml), where necessary. CVD 908-*htrA* and *S*. Paratyphi A 9150 and its derivatives were grown with 2x soy medium (20 g Hy-soy peptone, 10 g Hy-soy yeast extract, 3 g NaCl, ± 15 g of granulated agar (Difco) per liter) with guanine (0.001% v/v) and antibiotics added where necessary. Liquid cultures were incubated at 30°C or 37°C at 250 rpm for 16-24 hrs unless stated otherwise.

Modified minimal medium (MMM) used for complementation analysis was composed of M9 salts (K2HPO4, 7 g/l; KH2PO4, 3 g/l; (NH4)2SO4, 1 g/l (pH7.5)), 0.5% (w/v) casamino acids (Difco), 0.5% (w/v) glucose, 0.01% (w/v) MgSO4.7H20, 15 g of granulated agar (Difco) per liter and 1 µg/ml vitamin B1.

### 2. Plasmids and Molecular Genetic Techniques

Standard techniques were used for the construction of the plasmids represented here (see, for example, Sambrook et al., 1989). Plasmid extraction and gel purification of DNA fragments were performed using QIAprep Spin Miniprep and QIAquick Gel Extraction kits, respectively, as directed by the manufacturer (Qiagen Inc., Valencia, CA). Plasmids pCR-Blunt II-TOPO (Invitrogen, Carlsbad, CA), pGEM^{®}-T or pGEM^{®}-T Easy (Promega, Madison, WI) were used as intermediates for cloning blunt ended polymerase chain reaction (PCR) products generated with VentTM DNA Polymerase (New England Biolabs, Ipswich, MA). Plasmid pLowBlu 184 (E.M. Barry, unpublished data; CVD, University of Maryland, Baltimore) is a low copy number plasmid based on pACYC184 (ATCC) but containing the lactose operon sequence from pGEM^{®}-5Zf(+) (2767 - 273 bp; Promega, Madison, WI) in place of the tetracycline resistance gene between *Ava*I and *Hind*III*.* Taq-Pro™ DNA Polymerase (Denville Sci., Metuchen, NJ) was used for lambda Red-mediated mutagenesis, and for diagnostic PCR using 5 ul of a single bacterial colony diluted in 20 µl of sterile water. Taq-Pro™ DNA Polymerase was also used to add to pre-treat PCR fragments generated by VentTM DNA Polymerase prior to cloning into pGEM^{®}-T or pGEM^{®}-T Easy. All restriction enzymes were purchased from New England Biolabs. T4 DNA polymerase (NEB) was used to create blunt
ended DNA fragments. Electroporation of strains was performed in a Gene Pulser apparatus (Bio-Rad) set at 2.5 kV, 200 Ω, and 25 µF. Molecular weight markers used in DNA gel electrophoresis are O'GeneRuler™ 1 kb DNA Ladder, ready-to-use (#SM1163, Fermentas, Hanover, MD).

### 3. Lambda Red-Mediated Mutagenesis

This technique was performed as described by Datsenko and Wanner (Proc Natl Acad Sci USA. 2000 Jun 6;97(12):6640-50), with certain modifications. Briefly, 10 colonies of bacteria carrying Red helper plasmid pKD46 (reader is directed to the Datsenko and Wanner reference for more information about this plasmid) were added to 20 ml of 2x soy media supplemented with carbenicillin and L-arabinose (0.2%) and grown at 30°C, 250 rpm for 3 hrs (OD 600 nm of ∼ 0.6). Bacteria were made electrocompetent by washing 3 times with cold sterile water and concentrating 100 fold. Competent cells were electroporated with 100 ηg - 1 µg of gel-purified PCR product. Following electroporation, bacteria were repaired using 2x soy medium with or without guanine. Cells were incubated in 2x soy media at 37°C for 3 hrs prior to plating on 2x soy agar containing guanine and cml overnight. Antibiotic resistant colonies were selected and screened via PCR for alterations in the chromosomal regions of interest. Positive colonies were re-streaked onto 2x soy media containing cml, but lacking carbenicillin, to ensure loss of pKD46. Removal of the cml resistance cassette was performed as described by Datsenko and Wanner and involved using pCP20. Colonies exhibiting the desired genotype were re-streaked on 2x soy media lacking antibiotics to ensure the loss of the antibiotic resistance phenotype. Those selected for storage were re-screened via PCR prior to freezing at -70°C in 2x soy media containing 20% (v/v) glycerol.

### 4. Agglutination

S. Paratyphi A strains were tested with commercially available sera (Difco™ Salmonella O Antiserum Group A, Becton Dickson, Sparks, MD, lot # 4092221). Briefly, a small inoculum of bacteria taken from a fresh plate was emulsified in 20 µl of PBS on a glass slide. 5 µl of antisera was added, and the slide agitated gently until agglutination was observed. *S. flexneri* vaccine strain CVD 1208 (J Infect Dis. 2004 Nov 15;190(10):1745-54) or *E. coli* DH5 alpha served as negative control bacteria.

### 5. Assessment of virulence by intraperitoneal inoculation of mice

*Salmonella* virulence was assessed as described previously in Infect Immun. 2001 Aug;69(8):4734-41. Briefly, female BALB/c mice (Charles River Breeding Laboratories, Inc., Wilmington, Mass.) aged 6 to 8 weeks (three mice per group, three groups per vaccine strain) were injected intraperitoneally (i.p.) with various 10-fold dilutions of the bacteria (grown in the presence of guanine and antibiotics where necessary, and resuspended in phosphate-buffered saline PBS) mixed with 10% (wt/vol) hog gastric mucin (Difco, lot #4092018) in a final volume of 0.5 ml. Mice were monitored for extreme moribundity (close to death) or death every 24 hr for 72 h after inoculation. The 50% lethal dose (LD50) for each group of mice was calculated by linear regression analysis.

### 6. Construction of a deletion in guaBA.

The sequencing of the *S*. Paratyphi A genome was incomplete at the commencement of this project. Hence, all oligonucleotides primers and subsequent DNA templates for Lambda Red-mediated mutagenesis were constructed based on the annotated *S*. Typhi Ty2 genome sequence (Genbank accession number NC_004631, Dec. 16, 2004 version). Sequence comparison of the regions mutated in S. Paratyphi A with those of S. Typhi revealed greater than 99% DNA sequence identity.

The genes which encode inosine-5'-monophosphate dehydrogenase (*guaB*) and guanosine monophosphate synthetase (*guaA*) form an operon and are located at 414059 to 417178 bp on the *S*. Typhi Ty2 genome (SEQ ID NO:26; see also U.S. Patent No. 6,190,669 for detailed information with regard to the *guaBA* loci). Primers CVOL 13 and CVOL 15 (Table 1) bind to sequences outside the region designated for mutation. Primers CVOL 28 and CVOL 32 were designed to bind to regions of the Lambda Red template plasmid pKD3. The resulting PCR product encoded a cml resistance cartridge flanked on either side by a 100 bp of sequence homologous to *guaBA* at positions 413846 to 413945 (CVOL 28) and 417109 to 417010 (CVOL 32) on the *S*. Typhi Ty2 genome, respectively.

**Table 1**

| **Name** | **Sequence*^{a}*** | **SEQ ID NO:** | **Target** | **Region^{b}** |
|---|---|---|---|---|
| CVOL 13 | CTGCAGTCATTCCCACTCAATGGTAGC | 4 | Ty2 | 417176-417158 |
| CVOL 15 | GGAACATCGCACAGCGCA | 5 | Ty2 | 413715 - 413732 |
| CVOL 26 | GTGTAGGAGCTGCTTCG | 6 | pKD3 | 31-50 |
| CVOL 27 | CATATGAATATCCTCCTTAG | 7 | pKD3 | 1044-1025 |
| CVOL 28 | | 8 | pKD3 | 31-50 |
| | GCTGGAGCTGCTTC | | | |
| CVOL 32 | | 9 | pKD3 | 1044-1025 |
| CVOL 41 | GAAGGAGTATTGCCCATGCTACGTATCG | 10 | Ty2 | 414057 - 414077 |
| CVOL 64 | | 11 | Ty2 | 414057 - 414086 |
| CVOL 65 | | 12 | Ty2 | 417176-417155 |
| CVOL 85 | ACAGATAAACGCAAAGATGGCTCGGGCAAA | 13 | Ty2 | 2484865 - 2484836 |
| CVOL 86 | TTATTCGCCAGAAGCCTGCGCTTCCGGTTT | 14 | Ty2 | 2483597 - 2483626 |
| CVOL 87 | CCTGAGAATGGCATTTGCGTCGTCGTGTGC | 15 | Ty2 | 2484929 - 2484900 |
| CVOL 88 | ACGGCGTGTTTACAGGAAAAACGAAAGGGG | 16 | Ty2 | 2483520 - 2483549 |
| CVOL 89 | TCATACAGCGGAGAACGAGATAATTTGGCC | 17 | Ty2 | 2485740 - 2485711 |
| CVOL 90 | TTACATAAGTAAGTCACTGGGAGGCGCGCT | 18 | Ty2 | 2485027 - 2485056 |
| CVOL 91 | TCCATCAGGTTACAATCAGTACAGCAGATT | 19 | Ty2 | 2485800 - 2485771 |
| CVOL 92 | TCATTAGTATATACACAAAATCATTCGAGC | 20 | Ty2 | 2484961 - 2484990 |
| CVOL 122 | | 21 | Ty2 | 2485752 - 2485722 |
| CVOL 123 | | 22 | Ty2 | 2485024 - 2485056 |
| CVOL 124 | | 23 | Ty2 | 2484876 - 2484847 |
| CVOL 125 | | 24 | Ty2 | 2483597 - 2483626 |
| CVOL 128 | | 25 | Ty2 | 2485024 - 2485056 |

| | | | | |
|---|---|---|---|---|
| a. Primers are listed in 5' > 3' direction with restriction enzyme cleavage sites underlined. b. Indicates region of homology to *S*. Typhi Ty2 genome (genbank accession number NC_004631) or plasmid pKD3 (genbank accession number AY048742). | | | | |

*S*. Paratyphi A 9150 was made electrocompetent and transformed with pKD46, resulting in strain CV 250. Lambda Red mutagenesis was performed on CV 250 using the PCR product generated using primers CVOL 28 and CVOL 32 with template pKD3 containing a cml resistance marker (see the Datsenko and Wanner reference for more information about this plasmid). Transformants were plated at 37°C, and those exhibiting cml resistance were screened by PCR using CVOL 13 and CVOL 15. Unmodified *guaBA* amplified from *S*. Paratyphi A 9150 was found to be ∼ 3.5 kb (Figure 1, lane 1), whereas a ∼1.4 kb fragment was found in two clones with a mutated *guaBA* region (Figure 1, lanes 2 and 3). These mutants were named CV 411 and CV 412, respectively. Treatment of these mutants with pCP20 (see Datsenko and Wanner reference for more information about this plasmid) liberated the cml resistance cartridge. Four deletants were analyzed by PCR with primers CVOL 13 and CVOL 15 and found to have a ∼ 0.5 kb band (Figure 2, lanes 2 - 4) in comparison to a *guaBA::cml* progenitor (Figure 2, lane 1). Resulting *guaBA* deletants of *S.* Paratyphi A 9150 were named CV 415 - CV 418. The mutated *guaBA* region in CV 415 was PCR amplified with CVOL 15 and CVOL 13 and the product sequenced (polynucleotide sequence SEQ ID NO:1); the 5' and 3' regions of SEQ ID NO:1 are homologous to *guaBA,* whereas the center region is homologous to pKD3. Strain CV 415 was chosen for all subsequent studies.

### 7. In vitro complementation of the deletion in guaBA

*S*. Paratyphi A 9150 contains an undefined auxotrophy, making it incapable of growth on minimal medium without the addition of casamino acids. ATCC strain 11511 and a CVD *S*. Paratyphi A isolate 516 are also unable to grow on minimal medium, suggesting that they contain the same auxotrophy as found in *S*. Paratyphi A 9150.

To demonstrate that Lambda Red-mediated mutagenesis only targeted *guaBA* in CV 415, a pLowBlu 184-based (low copy number) plasmid was designed containing a minimal fragment encoding *guaBA* under the control of the lactose promoter (*P_{lacZ}*)*.* Primers CVOL 13 and CVOL 41 were used to amplify a ∼ 3.1 kb fragment encoding *guaBA* with an optimized ribosome binding site (GAAGGAG) 8 bp upstream of the start codon using the chromosome of CVD *908-htrA* as a template. This fragment was subcloned into pGEM^{®}-T Easy and excised with *Eco*RI*,* blunted with T4 DNA polymerase and cloned into the *Not*I site of pLowBlu 184 creating pATGguaBA (stored in CV 295).

CV 415 was electroporated with either pATGguaBA or pLowBlu 184 (control) and plated on 2x Soy media containing guanine and cml, creating strains CV 486 and CV 487, respectively. 2x soy medium lacking guanine is able to support the growth of *guaBA* deleted *S*. Paratyphi A. Single colonies of each were re-streaked onto MMM containing cml and incubated at 37°C overnight. As shown in Figure 3, CV 486 was able to grow on MMM (plate 2) in contrast to the control (CV 487; plate 1) indicating the minimal fragment cloned into pATGguaBA was able to complement the deletion of *guaBA* from the chromosome of CV 415.

### 8. Construction of secondary deletions in CV 415

In order to minimize the reversion of *guaBA* deleted *S*. Paratyphi A 9150 to a wild type (wt) genotype, additional genes were targeted as secondary attenuating markers. These genes were *clpP* and *clpX,* which encode a serine-protease and a chaperone ATPase, respectively (reviewed in Structure (Camb). 2004 Feb;12(2):175-83). Disruption of *clpP* and/or *clpX* has been shown to significantly reduce the colonization potential of *Salmonella* Typhimurium in mice (Infect Immun. 2001 May;69(5):3164-74). In *S*. Typhi Ty2, *clpX* (SEQ ID NO:39) and *clpP* (SEQ ID NO:40) are both located between 2483597 to 2485743 (SEQ ID NO:27) on the complementary strand of the chromosome, respectively, and are expressed from individual promoters. Wt S. Paratyphi A 9150 was also subjected to mutagenesis such that the virulence of mutants containing single deletions in either *clpX* or *clpP* could be assessed in mice.

To delete *clpX,* primers CVOL 85 and 86 were designed to amplify a ∼ 1.3 kb fragment encoding *clpX* lacking a start codon from CVD 908-htrA. This fragment was column purified, Taq-Pro™ DNA Polymerase treated and cloned into pGEM^{®}-T (stored in CV 472). The resulting vector, pGEM^{®}-T::*clpX* was digested with *Nru*I and *Eco*RI to remove a ∼ 0.9 kb fragment, and treated with T4 DNA polymerase to create blunt ends. A cml cartridge isolated from pCR-Blunt II-TOPO as an *Eco*RI fragment was blunted and inserted into pGEM^{®}-T::*clpX*. This cml cartridge had been previously created by PCR using CVOL 25 and CVOL 26 with pKD3 as a template (stored in CV 134). Following ligation and transformation, PCR was used with primers CVOL 26 and CVOL 85 to confirm insertion of the cml cartridge in the correct orientation for Lambda Red mutagenesis. A positive clone was identified, named pGEM^{®}-T::(*clpX*::cm1) and stored as CV 481.

*guaBA* deleted *S*. Paratyphi A (CV 415) was transformed with pKD46 and stored as CV 421. CV 421 and CV 250 (wt S. Paratyphi A 9150 transformed with pKD46) were subjected to Lambda Red mutagenesis with a ∼ 1.4 kb PCR product amplified from pGEM^{®}-T::(*clpX*::cml) using CVOL 85 and CVOL 86. Cml resistant mutants were isolated and screened by PCR with CVOL 87 and CVOL 88, which bind to regions outside those homologous to CVOL 85 and CVOL 86. Mutants exhibiting a correct PCR profile were selected for treatment with pCP20 to remove the cml cartridge. Figure 4 shows that mutants containing an altered *clpX* gene exhibited a smaller ∼ 0.6 kb band by PCR (Panel A, lanes 1 - 6) compared to that found in unaltered *S*. Paratyphi A 9150 (Panel A, lane 7). PCR analysis of the same strains with CVOL 13 and 15 confirmed that *guaBA* was deleted only from strains derived from CV 415 and not CV 250 (Panel B, lanes 4 - 6, compared to lanes 1 - 3 and 7). Mutants lacking *clpX,* and both *clpX* and *guaBA,* were stored as CV 532 and CV 534, respectively. The mutated *clpX* region in CV 532 and CV 534 was PCR amplified with primers CVOL 87 and CVOL 88 and the product sequenced (SEQ ID NO:2); the 5' and 3' regions of SEQ ID NO:2 are homologous to *clpX,* whereas the center region is homologous to pKD3.

To delete *clpP,* CVOL 89 and 90 were designed to amplify a ∼ 0.7 kb fragment encoding *clpP* lacking a start codon from CVD 908-htrA. This fragment was column purified and cloned into pGEM^{®}-T, creating pGEM®-T::*clpP* (stored as CV 470). pGEM^{®}-*T::clpP* was digested with *Pst*I and *Nsi*I*,* T4 DNA polymerase treated and religated (creating pGEM^{®}-T::*clpP*m, stored as CV 484) in order the remove *Nde*I and *Hinc*II sites from the vector backbone. pGEM^{®}-T::*clpP*m was then digested with *Nde*I and *Hinc*II to remove DNA fragments totaling ∼ 0.5 kb in size, and T4 DNA polymerase treated. Similarly to that abovementioned, a cml cartridge isolated from pCR-Blunt II-TOPO as an EcoRI fragment was T4 DNA polymerase treated and used to replace the fragments removed from pGEM^{®}-*T::clpPm.* Following ligation and transformation, PCR was used with primers CVOL 26 and CVOL 85 to confirm insertion of the cml cartridge in the correct orientation for Lambda Red mutagenesis. A positive clone was identified, named pGEM^{®}-T::(*clpP*m::cml) and stored as CV 501.

Wt and *guaBA* deleted *S*. Paratyphi A 9150 containing pKD46 (CV 250 and CV 421, respectively) were subjected to Lambda Red mutagenesis with a ∼ 1.4 kb PCR product amplified from pGEM^{®}-T::(*clpP*m::cml) using CVOL 89 and CVOL 90. Cml resistant mutants were isolated and screened by PCR with CVOL 91 and CVOL 92, which bind to regions outside those homologous to CVOL 89 and CVOL 90. Mutants exhibiting a correct PCR profile were selected for treatment with pCP20 to remove the cml cartridge.

Figure 5 shows that mutants containing an altered *clpP* gene exhibited a smaller ∼ 0.4 kb band by PCR (Panel A, lanes 1 - 6) compared to that found in unaltered S. Paratyphi A 9150 (Panel A, lane 7). PCR analysis of the same strains using primers CVOL 13 and 15 confirmed that *guaBA* was deleted only from strains derived from CV 415 and not from those based on CV 250 (Panel B, lanes 4 - 6, compared to lanes 1 - 3 and 7). Mutants lacking *clpP,* and both *clpP* and guaBA, were stored as CV 528 and CV 530, respectively. The mutated *clpP* region in CV 528 and CV 530 were PCR amplified with primers CVOL 87 and CVOL 88 and the product sequenced (polynucleotide sequence SEQ ID NO:3); the 5' and 3' regions of SEQ ID NO:3 are homologous to *clpP,* whereas the center region is homologous to pKD3.

### 9. Construction of low copy plasmids for complementation analysis

As performed above, to confirm that the Lambda Red-mediated mutagenesis targeted only specific loci, mono- or bi-cistronic pLowBlu 184-based (low copy number) plasmids were designed containing minimal fragments encoding either *clpX* or *clpP,* or *guaBA* immediately downstream of either *clpX* or *clpP.* Constitutive expression of the genes in these plasmids was directed by P*_{lacZ}.*

Primers CVOL 64 and CVOL 65 were used to PCR amplify *guaBA* from CVD 908-*htrA* containing an enhanced ribosome binding site with *Nde*I and *Nsi*I restriction sites at the 5' and 3' ends, respectively. The product was column purified and ligated into pCR-Blunt II-TOPO (stored as CV 394), extracted as a ∼ 3.5 kb *Nde*I *- Nsi*I fragment and cloned into the *Nde*I and *Nsi*I sites in pLowBlu 184, creating pguaBAV.2 (stored as CV 482).

To create a low copy number plasmid encoding *clpP,* primers CVOL 122 and CVOL 123 were used to amplify *clpP* with an enhanced ribosome binding site from CVD *908-htrA* with *Not*I and *Nru*I sites at the 5' and 3' ends, respectively. The ∼ 0.7 kb product was column purified and ligated into pCR-Blunt II-TOPO (stored as CV 567), extracted as a *Not*I *- Nru*I fragment and ligated into pLowBlu 184 previously cut with *Not*I and *Nde*I*,* creating pATGclpP (stored as CV 584). To construct a bi-cistronic plasmid containing *clpP* and *guaBA,* primers CVOL 122 and CVOL 128 were designed to amplify *clpP* with an enhanced ribosome binding site from CVD *908-htrA* with *Not*I sites at both the 5' and 3' ends. The ∼ 0.7 kb product was column purified and ligated into pCR-Blunt II-TOPO (stored as CV 600), extracted as a *Not*I fragment and ligated into pguaBAV.2 previously cut with *Not*I*,* creating pATGclpPATGguaBA (stored as CV 603).

To create a low copy plasmid encoding *clpX,* primers CVOL 124 and CVOL 125 were used to amplify *clpX* with an enhanced ribosome binding site from CVD *908-htrA* with *Not*I and *Nde*I sites at the 5' and 3' ends, respectively. The ∼ 1.3 kb product was column purified and ligated into pCR-Blunt II-TOPO (stored as CV 569), extracted as a *Not*I *- Nde*I fragment and ligated into either pLowBlu 184 or pguaBAV.2 both previously cut with *Not*I and *Nde*I*,* creating pATGclpX (stored as CV 582) and pATGclpXATGguaBA (stored as CV 573).

### 10. Assessment of virulence of S. Paratyphi A and mutants in mice

LD₅₀ studies were performed to compare the virulence of wt S. Paratyphi A 9150 with that of each mutant in intraperitoneally injected mice.

Figure 6 shows the LD₅₀ data obtained with the injection of mice with wt and *guaBA* deleted *S*. Paratyphi A. Wt S. Paratyphi A has an LD₅₀ value of < 10 bacteria per mouse. In contrast, *guaBA* deleted S. Paratyphi A had a LD₅₀ value ∼ 4.5 logs greater. Complementation of the *guaBA* mutant with pLowBlu 184 did not alter the LD₅₀ value, whereas transformation with pATGguaBA restored wt-like virulence.

Figure 7 shows the LD₅₀ data obtained with the injection of mice with wt S. Paratyphi A, *clpX*-deleted *S*. Paratyphi A, or *clpX-guaBA*-deleted *S*. Paratyphi A. The LD₅₀ values of wt and *guaBA*-deleted *S*. Paratyphi A were consistent with those achieved previously. *clpx*-deleted *S.* Paratyphi A displayed a ∼ 1 log greater LD₅₀ value as compared to the *guaBA* mutants, indicating that a deletion in *clpX* was not as attenuating as that in *guaBA.* Complementation of the *clpX-*deleted or the *clpX-guaBA* deleted strains with pLowBlu 184 had no effect, whereas transformation with pATGclpX and pATGclpXATGguaBA, respectively, restored wt-like virulence.

Figure 8 shows the LD₅₀ data of mice injected with wt *S*. Paratyphi A, *clpP*-deleted *S*. Paratyphi A or *clpP-guaBA*-deleted *S*. Paratyphi A. The LD₅₀ values of wt and *guaBA-*deleted *S*. Paratyphi A were consistent to those achieved previously. As with the *clpX-*deleted strain, *clpP-*deleted *S*. Paratyphi A exhibited increased virulence as compared to the *guaBA* mutant. This indicated that a deletion in *clpP* was not as attenuating as that in *guaBA.* Complementation of the *clpX* or the *clpX-guaBA*-deleted strains with pLowBlu 184 had no effect on virulence. Transformation of these strains with pATGclpP and pATGclpPATGguaBA, respectively, did not completely restore wt virulence. Without being bound by any theory, regulated expression of *clpP,* as opposed to unregulated expression as encoded by pATGclpP and pATGclpPATGguaBA, is required to fully complement the *clpP* mutation.

### 11. S. Paratyphi A derived conjugate vaccine

Conjugate vaccines are developed and tested as described herein. A conjugate vaccine of the present invention comprises O polysaccharide (OPS) of *S*. Paratyphi A and a flagellin protein of *S*. Paratyphi A. In addition, preferably a conjugate vaccine of the present invention consists of OPS of *S*. Paratyphi A and a flagellin protein of *S*. Paratyphi A. Even more preferably, a conjugate vaccine of the present invention consists essentially of OPS of *S*. Paratyphi A and a flagellin protein of *S*. Paratyphi A.

Disclosed herein is a conjugate vaccine comprising a flagellin protein comprises any known flagellin protein (see, for example, International patent publication WO 2005/042564). In particular any known flagellin protein of *S*. Paratyphi A. In the present invention, the flagellin protein is phase 1 flagellin (fliC).

A conjugate vaccine comprising OPS comprises any known OPS of S. Paratyphi A. In particular aspects of the invention, OPS belongs to Group A OPS which by definition exhibits immunodominant epitope 2 and usually also has non-dominant epitopes 1 and 12. The Salmonella Group A OPS contains a backbone of repeats of mannosyl-rhamnosyl-galactose to which the dideoxyhexose sugar paratose is linked alpha1,3 to the mannose residue. This gives rise to the immunodominant epitope "2" that defines the OPS of Salmonella Group A.

A broad-based vaccine against paratyphoid and typhoid fever is prepared by including conjugates to prevent *S*. Paratyphi B disease, *S*. Paratyphi C disease, and *S*. Typhi disease. The conjugates are made according to the same methods and strategies described herein of linking the relevant purified OPS to the homologous phase 1 flagellin protein. Thus, an *S*. Paratyphi B conjugate vaccine comprises, consists of, or consists essentially of Group B OPS conjugated to phase 1 flagella protein "b" (i.e., to the protein subunit encoded by fliC of *S*. Paratyphi B). An *S*. Paratyphi C conjugate vaccine comprises, consists of, or consists essentially of Group C1 OPS conjugated to phase 1 flagella protein "c" (i.e., conjugated to the protein subunit encoded by fliC of *S*. Paratyphi C). An *S*. Typhi conjugate vaccine comprises, consists of, or consists essentially of Group D OPS conjugated to phase 1 flagella protein "d" (i.e., conjugated to the protein subunit encoded by fliC of *S*. Typhi). In addition to the above-described conjugates being made according to the same methods and strategies, these conjugates are also administered and manufactured according the same methods and strategies described herein.

Conjugation of OPS of *S*. Paratyphi A and a flagellin protein of *S*. Paratyphi A can be carried out by methods known to one of ordinary skill in the art (see, for example, US Patent Application Publication No. 20090028889). In particular aspects of the invention drawn to conjugation of OPS of *S*. Paratyphi A and a flagellin protein of *S*. Paratyphi A, methods for the chemical conjugation of polypeptides, carbohydrates, and/or lipids are well known in the art (see, for example, Hermanson. Bioconjugate Techniques (Academic Press; 1992); Aslam and Dent, eds. Bioconjugation: Protein coupling Techniques for the Biomedical Sciences (MacMillan: 1998); and Wong Chemistry of Protein Conjugation and Cross-linking (CRC Press: 1991)). For example, in the case of carbohydrate or lipid conjugation, functional amino and sulfhydryl groups may be incorporated therein by conventional chemistry. For instance, primary amino groups may be incorporated by reaction with ethylenediamine in the presence of sodium cyanoborohydride and sulfhydryls may be introduced by reaction of cysteamin dihydrochloride followed by reduction with a standard disulfide reducing agent.

Heterobifunctional crosslinkers, such as, for example, sulfosuccinimidyl (4-iodoacetyl) aminobenzoate, which link the epsilon amino group on the D-lysine residues of copolymers of D-lysine and D-glutamate to a sulfhydryl side chain from an amino terminal cysteine residue on the peptide to be coupled, may be used to increase the ratio of OPS of *S*. Paratyphi A to a flagellin protein of *S*. Paratyphi A and/or a flagellin protein of *S*. Paratyphi A. to OPS of *S*. Paratyphi A in the conjugate.

OPS of *S*. Paratyphi A or a flagellin protein of *S*. Paratyphi A may contain amino acid side chains such as amino, carbonyl, hydroxyl, or sulfhydryl groups or aromatic rings that can serve as sites for conjugation. Residues that have such functional groups may be added to either OPS of *S.* Paratyphi A or to a flagellin protein of *S.* Paratyphi A. Such residues may be incorporated by solid phase synthesis techniques or recombinant techniques, both of which are well known in the art.

OPS of *S.* Paratyphi A and a flagellin protein of *S.* Paratyphi A may be chemically conjugated using conventional crosslinking agents such as carbodiimides. Examples of carbodiimides are 1-cyclohexyl-3-(2-morpholinyl-(4-ethyl) carbodiimide (CMC), 1-ethyl-3-(3-dimethyaminopropyl) carbodiimide (EDC), and 1-ethyl-3-(4-azonia-44-dimethylpentyl) carbodiimide.

Examples of other suitable crosslinking agents are cyanogen bromide, glutaraldehyde and succinic anhydride. In general, any of a number of homobifunctional agents including a homobifunctional aldehyde, a homobifunctional epoxide, a homobifunctional imidoester, a homobifunctional N-hydroxysuccinimide ester, a homobifunctional maleimide, a homobifunctional alkyl halide, a homobifunctional pyridyl disulfide, a homobifunctional aryl halide, a homobifunctional hydrazide, a homobifunctional diazonium derivative or a homobifunctional photoreactive compound may be used. Also included are heterobifunctional compounds, for example, compounds having an amine-reactive and a sulfliydryl-reactive group, compounds with an amine-reactive and a photoreactive group, and compounds with a carbonyl-reactive and a sulflzydryl-reactive group.

Specific examples of homobifunctional crosslinking agents include the bifunctional N-hydroxysuccinimide esters dithiobis (succinimidylpropionate), disuccinimidyl suberate, and disuccinimidyl tartarate; the bifunctional imidoesters dimethyl adipimidate, dimethyl pimelimidate, and dimethyl suberimidate; the bifunctional sulfhydryl-reactive crosslinkers 1,4-di-[3'-(2'-pyridyldithio) propion-amido]butane, bismaleimidohexane, and bis-N-maleimido-1,8-octane; the bifunctional aryl halides 1,5-difluoro-2,4-dinitrobenzene and 4,4'-difluoro-3,3'-dinitrophenylsulfone; bifunctional photoreactive agents such as bis-[b-(4-azidosalicylamide)ethyl]disulfide; the bifunctional aldehydes formaldehyde, malondialdehyde, succinaldehyde, glutaraldehyde, and adiphaldehyde; a bifunctional epoxied such as 1,4-butaneodiol diglycidyl ether; the bifunctional hydrazides adipic acid dihydrazide, carbohydrazide, and succinic acid dihydrazide; the bifunctional diazoniums o-tolidine, diazotized and bis-diazotized benzidine; the bifunctional alkylhalides N1N'-ethylene-bis(iodoacetamide), N1N'-hexamethylene-bis(iodoacetamide), N1N'-undecamethylene-bis(iodoacetamide), as well as benzylhalides and halomustards, such as ala'-diiodo-p-xylene sulfonic acid and tri(2-chloroethyl)amine, respectively.

Examples of other common heterobifunctional crosslinking agents that may be used include, but are not limited to, SMCC (succinimidyl-4-(N-maleimidomethyl)cyclohexane-1-carboxylate), MBS (m-maleimidobenzoyl-N-hydroxysuccinimide ester), SIAB (N-succinimidyl(4-iodacteyl) aminobenzoate), SMPB (succinimidyl-4-(p-maleimidophenyl)butyrate), GMBS (N-(-maleimidobutyryloxy)succinimide ester), MPHB (4-(4-N-maleimidopohenyl) butyric acid hydrazide), M2C2H (4-(N-maleimidomethyl)cyclohexane-1-carboxyl-hydrazide), SMPT (succinimidyloxycarbonyl-a-methyl-a-(2-pyridyldithio)toluene), and SPDP (N-succinimidyl 3-(2-pyridyldithio) propionate). For example, crosslinking may be accomplished by coupling a carbonyl group to an amine group or to a hydrazide group by reductive amination.

In another aspect of the invention, OPS of *S.* Paratyphi A and a flagellin protein of *S.* Paratyphi A are conjugated through polymers, such as PEG, poly-D-lysine, polyvinyl alcohol, polyvinylpyrollidone, immunoglobulins, and copolymers of D-lysine and D-glutamic acid. Conjugation of OPS of *S.* Paratyphi A to a flagellin protein of *S.* Paratyphi A may be achieved in any number of ways, including involving one or more crosslinking agents and functional groups on the OPS of *S.* Paratyphi A and/or a flagellin protein of *S.* Paratyphi A. The polymer may be derivatized to contain functional groups if it does not already possess appropriate functional groups.

Without being bound by theory, a conjugate vaccine of the invention made with OPS will stimulate bactericidal antibodies (Konadu E et al. Infect Immun 1996; 64(7):2709-15; Maclennan et al. J Clin.Invest. 2008; 118:1553-62.) Using a flagellin protein as the carrier will result in enhanced serum O antibody responses because of the adjuvant (TLR5 agonist) effect of the flagellin protein (Feuillet et al. PNAS 2006; 103:12487-92; Gewirtz et al. J.Immunol. 2001; 167:1882-5; Huleatt et al. Vaccine. 2007; 25:763-75).

### 12. Vaccination Comprising a Conjugate Comprising OPS and a Flagellin Protein

Vaccine strategies are well known in the art and therefore the vaccination strategy encompassed by the invention does not limit the invention in any manner. Disclosed herein, the conjugate vaccine is administered alone in a single application or administered in sequential applications. Disclosed herein, the conjugate vaccine is administered as a component of a homologous or heterologous prime/boost regimen. In particular aspects disclosed herein drawn to heterologous prime/boost, a mucosal prime/parenteral boost immunization strategy is used. For example, an attenuated *S.* Paratyphi A strain as taught herein is administered orally and subsequently boosted parentally with a conjugate vaccine of OPS of S. Paratyphi A and a flagellin protein of S. Paratyphi A as described herein.

### 13. Methods of large-scale manufacture of a Flagellin Protein derived from S.

### Paratyphi A

When generating attenuated vaccines for administration or for components used to make a conjugation vaccine (including, for example, OPS and a flagellin protein), attention to the degree of attenuation must be considered. In regard to large-scale manufacturing, attenuation is particularly important so as to ensure safety in the manufacturing process (including, for example, environmental safety). Manufacturing of an attenuated S. Paratyphi A for use in the invention is sufficiently attenuated to ensure safety of not only administration of the strain, but also manufacturing of the strain for the production of *S.* Paratyphi A to derive components for making a conjugate vaccine.

In particular aspects of the invention drawn to the *S.* Paratyphi A to derive components for making a conjugate vaccine, the attenuated strain produces, for example, more flagella than wild-type *S.* Paratyphi A or causes the export of a flagellin protein from *S.* Paratyphi A.

In certain aspects of the invention, an attenuated strain that having increased flagella compared to a wild-type strain is particularly advantageous from at least a manufacturing perspective. In particular aspects of the invention, an attenuated *S.* Paratyphi A strain having one or more mutations described herein leading to attenuation and hyperflagellation can be used for manufacturing an oral vaccine and/or components for a conjugate vaccine. For example, an attenuated strain of *S.* Paratyphi A having a mutation in the guaBA loci, the guaB gene, or the guaA gene, and mutation in the clpP gene produces an attenuated and a phenotypically hyperflagellated *S.* Paratyphi A strain (see, for example, Figure 9). Methods of purification of a flagellin protein from whole flagella are known in the art or can be readily modified by one of ordinary skill in the art using methods know in the art. For example, by modifying the method of Ibrahim et al., purification of flagella is achieved; below pH 3.0, flagella dissociate into flagellin subunits (Ibrahim et al. J.Clin.Microbiol. 1985; 22:1040-4. Modern purification methods and clpP mutants should markedly increase yield and purity of a flagellin protein used to construct a conjugate vaccine of the invention (see, for example, Ogushi et al. J.Biol.Chem. 2001; 276:30521-6; Yoon et al. Infect.Immun. 2008; 76:1282-8).

In other aspects of the invention, export of a flagellin protein from an attenuated *S.* Paratyphi A strain is used to derive a flagellin protein used to construct a conjugate vaccine of the invention. For example, a mutation in the fliD gene, flgL gene, or flgK gene causes export of flagellin monomers into the supernatant. Disclosed herein, an attenuated *S.* Paratyphi A strain has a mutation in at least the guaBA loci, the guaB gene, or the guaA gene, and mutation in the fliD gene, flgL gene, or flgK gene.

Disclosed herein, *S.* Paratyphi A containing a guaBA mutation and a fliD mutation is made and used to derive a flagellin protein used to construct a conjugate vaccine of the invention. The mutation in guaBA has been described herein. The mutation of the fliD gene can be accomplished by a number of methods, including for example, knocking out the fliD gene. A method used to knockout fliD includes, for example, referencing Table 2, PCR #1 mixed with PCR #2 and then amplified with primers 5FliD and 3FRT-aph in an overlapping PCR, which generates a kanomycin-resistant fragment that is recovered in, for example, PSMART-LCAMP TM vector (Lucigen). PCR #3 can also be recovered in PSMART-LCAMP TM vector, then cut out with BamHI and XhoI, and inserted into the pSMART-fliD-aph clone cut with BamHI and XhoI. The final assembled fragment is cut out and electroporated into S. Paratyphi A carrying pKD46 and selection for kanomycin resistence according to the method of Datsenko and Wanner, followed by crossing out the aph cassette, where applicable, using pCP20 according to the method of Datsenko and Wanner (Proc Natl Acad Sci USA. 2000 Jun 6;97(12):6640-50).

**Table 2**

| | | |
|---|---|---|
| PCR #1 (using Salmonella guaBA chromosomal template DNA) | 5FliD: 5'-tcacgcacacgctgcaggttgttgttgatttc-3' | SEQ ID NO:41 |
| | 5FliD-rev: 5'-gaacttcGAAGCAGCTCCAGCacctaat gatgaaattgaagccatgc-3' | SEQ ID NO:42 |
| PCR #2 (using pKD 13 template DNA) | 5FRT-aph: 5'-GCTGGAGCTGCTTCgaagttc-3' | SEQ ID NO:43 |
| | 3FRT-aph: 5'-ctcgagTTCCGGGGATCCGTCGAC CTGCAGTTC-3' | SEQ ID NO:44 |
| PCR #3 (using Salmonella guaBA chromosomal template DNA) | 3FliD: 5'-GGATCCgctatgaacaagtcctgataacagaggt-3' | SEQ ID NO:45 |
| | 3FliD-rev: 5'-CTCGAGttaacgagactcctggaaagatgcttt cggtgaaatctgc-3' | SEQ ID NO:46 |

In addition to the attenuated S. Paratyphi A strains described herein for vaccination purposes and for deriving a flagellin protein used as a component of the conjugate vaccine described herein, these strains can be used to derive OPS that is used as a component of the conjugate vaccine described herein. Disclosed herein is any of the foregoing mutations in any combination that provides for maximum efficacy or manufacturing results (e.g., any combination of mutations in guaBA loci, the guaB gene, guaA gene, clpP gene, clpX gene, fliD gene, flgL gene, flgK gene, or any other mutation described herein can be made in all possible combinations or variations).

### Cited Documents

Levine MM, Herrington D, Murphy JR, Morris JG, Losonsky G, Tall B, Lindberg A, Svenson S, Baqar S, Edwards MF, Stocker B. Safety, infectivity, immunogenicity and in vivo stability of two attenuated auxotrophic mutant strains of Salmonella Typhi, 541Ty and 543Ty, as live oral vaccines in man. J Clin Invest 79:888-902, 1987.

Levine MM, Ferreccio C, Black RE, Chilean Typhoid Committee, Germanier R. Large-scale field trial of Ty21a live oral typhoid vaccine in enteric-coated capsule formulation. Lancet I:1049-1052, 1987.

Ferreccio C, Levine MM, Rodriguez H, Contreras R, Chilean Typhoid Committee. Comparative efficacy of two, three, or four doses of Ty21A live oral typhoid vaccine in enteric-coated capsules: a field trial in an endemic area. J Infect Dis 159:766-769, 1989.

Levine MM, Ferreccio C, Black RE, Tacket CO, Germanier R. Progress in vaccines against typhoid fever. Rev Infect Dis 2 (supplement 3):S552-S567, 1989.

Van de Verg L, Herrington DA, Murphy JR, Wasserman SS, Formal SB, Levine MM. Specific IgA secreting cells in peripheral blood following oral immunization with bivalent Salmonella Typhi/Shigella sonnei vaccine or infection with pathogenic S. sonnei in humans. Infect Immun 58:2002-2004, 1990.

Levine MM, Hone D, Heppner DG, Noriega F, Sriwathana B. Attenuated Salmonella as carriers for the expression of foreign antigens. Microecology and Therapy 19:23-32, 1990.

Herrington DA, Van De Verg L, Formal SB, Hale TL, Tall BD, Cryz SJ, Tramont EC, Levine MM. Studies in volunteers to evaluate candidate Shigella vaccines: further experience with a bivalent Salmonella Typhi-Shigella sonnei vaccine and protection conferred by previous Shigella sonnei disease. Vaccine 8:353-357, 1990.

Cryz SJ, Levine MM, Kaper JB. Randomized double-blind placebo-controlled trial to evaluate the safety and immunogenicity of the live oral cholera vaccine strain CVD 103-HgR in adult Swiss. Vaccine 8:577-580, 1990.

Levine MM, Ferreccio C, Cryz S, Ortiz E. Comparison of enteric-coated capsules and liquid formulation of Ty21a typhoid vaccine in a randomized controlled field trial. Lancet 336:891-894, 1990.

Servos S, Chatfield S, Hone D, Levine MM, Dimitriadis G, Pickard D, Dougan G, Fairweather N, Charles I. Molecular cloning and characterization of the aroD gene encoding 3-dehydroguinase from Salmonella Typhi. J Gen Micro 137:147-152, 1990.

Levine MM, Hone D, Tacket C, Ferreccio C, Cryz S. Clinical and field trials with attenuated Salmonella Typhi as live oral vaccines and as "carrier vaccines". Res Microbiol 141:807-816, 1990.

Black RE, Levine MM, Ferreccio C, Clements ML, Lanata C, Rooney J, Germanier R, and the Chilean Typhoid Committee. Efficacy of one or two doses of Ty21a Salmonella Salmonella Typhi vaccine in enteric-coated capsules in a controlled field trial. Vaccine 8:81-84, 1990.

Tacket CO, Losonsky G, Taylor DN, Baron L, Kopeck D, Cryz S, Levine MM. Lack of immune response to the Vi component of a Vi-positive variant of the Salmonella Typhi live oral vaccine strain Ty21a in volunteer studies. J Infect Dis 163:901-904, 1991.

Hone DM, Harris AM, Chatfield S, Dougan G, Levine MM. Construction of genetically-defined double aro mutants of Salmonella Typhi. Vaccine 9:810-816, 1991.

Tacket CO, Hone DM, Curtiss R III, Kelly SM, Losonsky G, Guers L, Harris AM, Edelman R, Levine MM. Comparison of the safety and immunogenicity of ΔaroC ΔaroD and Δcya Δcrp Salmonella typhi strains in adult volunteers. Infect Immun 60:536-541, 1992.

Tacket CO, Hone DM, Losonsky G, Guers L, Edelman R, Levine MM. Clinical acceptability and immunogenicity of CVD 908 Salmonella Typhi vaccine strain. Vaccine 10:443-446, 1992.

Hone DM, Tacket CO, Harris AM, Kay B, Losonsky G, Levine MM. Evaluation in volunteers of a candidate live oral attenuated Salmonella Typhi vaccine. J Clin Invest 90:412-420, 1992.

Olanratmanee T, Levine MM, Losonsky GA, Thisyakorn U, Cryz SJ Jr. Safety and immunogenicity of Salmonella Typhi Ty21a liquid formulation vaccine in 4- to 6-year-old Thai children. J Infect Dis 166:451-452, 1992.

Chatfield SN, Fairweather N, Charles I, Pickard D, Levine MM, Hone D, Posada M, Strugnell RA, Dougan G. Construction of a genetically defined Salmonella Typhi Ty2 aroA, aroC mutant for the engineering of a candidate live oral typhoid-tetanus vaccine. Vaccine 10:8-11, 1992.

Cryz SJ Jr, Vanprapar N, Thisyakorn U, Olanratamanee T, Losonsky G, Levine MM, Chearskul S. Safety and immunogenicity of Salmonella Typhi Ty21a vaccine in young children. Infect Immun 61:1149-115, 1993.

Gonzalez C, Hone D, Noriega F, Tacket CO, Davis JR, Losonsky G, Nataro JP, Hoffman S, Malik A, Nardin E, Sztein MB, Heppner DG, Fouts TR, Isibasi A, Levine MM. Salmonella Typhi strain CVD 908 expressing the circumsporozoite protein of Plasmodium falciparum: strain construction, safety and immunogenicity. J Infect Dis 169:927-931, 1994.

Sztein MB, Wasserman SS, Tacket CO, Edelman R, Hone D, Lindberg AA, Levine MM. Cytokine production patterns and lymphoproliferative responses in volunteers orally immunized with attenuated vaccine strains of Salmonella Typhi. J Infect Dis 170:1508-1517, 1994.

Hone DM, Harris AM, Lim V, Levine MM. Construction and characterization of isogenic O-antigen variants of Salmonella Typhi. Molec Microbiol 13:525-530, 1994.

Pickard D, Li J, Roberts M, Maskell D, Hone D, Levine M, Dougan G, Chatfield S. Characterization of defined ompR mutants of Salmonella Typhi: ompR is involved in the regulation of Vi polysaccharide expression. Infect Immun 62:3984-3993, 1994.

Noriega FR, Wang JY, Losonsky G, Maneval DR, Hone DM, Levine MM. Construction and characterization of attenuated ΔaroA ΔvirG Shigella flexneri 2a strain CVD 1203, a prototype live oral vaccine. Infect Immun 62:5168-5172, 1995.

Gómez-Duarte, OG, Galen J, Chatfield SN, Rappuoli R, Eidels L, Levine MM. Expression of fragment C of tetanus toxin fused to a carboxyl-terminal fragment of diphtheria toxin in Salmonella Typhi CVD 908 vaccine strain. Vaccine 13:1596-1602, 1995.

Cryz SJ Jr, Que JU, Levine MM, Wiedermann G, Kollaritsch H. Safety and immunogenicity of a live oral bivalent typhoid fever (Salmonella Typhi-Ty21a) cholera (Vibrio cholerae CVD 103-HgR) vaccine in healthy adults. Infect Immun 63:1336-1339, 1995.

Sztein MB, Tanner MK, Polotsky Y, Orenstein JM, Levine MM. Cytotoxic T lymphocytes after oral immunization with attenuated strains of Salmonella Typhi in humans. J Immunol 155:3987-3993, 1995.

Levine MM, Galen J, Barry E, Noriega F, Chatfield S, Dougan G, Tacket C. Attenuated Salmonella as live oral vaccines against typhoid fever and as live vectors. J Biotechnology 44:193-196, 1995.

Noriega FR, Losonsky G, Wang JY, Formal SB, Levine MM. Further characterization of ΔaroA, ΔvirG Shigella flexneri 2a strain CVD 1203 as a mucosal Shigella vaccine and as a live vector for delivering antigens of enterotoxigenic Escherichia coli. Infect Immun 64:23-27, 1996.

Noriega FR, Losonsky G, Lauderebaugh C, Liao FM, Wang JY, Levine MM. Engineered ΔguaBA ΔvirG Shigella flexneri 2a strain CVD 1205: construction, safety, immunogenicity, and potential efficacy as a mucosal vaccine. Infect Immun 64:3055-3061, 1996.

Kotloff K, Noriega F, Losonsky G, Sztein MB, Nataro JP, Levine MM. Safety, immunogenicity and transmissibility in humans of CVD 1203, a live oral Shigella flexneri 2a vaccine candidate attenuated by deletions in aroA and virG. Infect Immun 64:4542-4548, 1996.

Barry EM, Gomez-Duarte O, Chatfield S, Rappuoli R, Losonsky GA, Galen JE, Levine MM. Expression and immunogenicity of pertussis toxin S1 subunit-tetanus toxin fragment C fusions in Salmonella Typhi vaccine strain CVD 908. Infect Immun 64:4172-4181,1996.

Tacket CO, Sztein MB, Losonsky GA, Wasserman SS, Nataro JP, Edelman R, Galen JE, Pickard D, Dougan G, Chatfield SN, Levine MM. Safety of live oral Salmonella Typhi vaccine strains with deletions in htrA and aroC aroD and immune response in humans. Infect Immun 65:452-456, 1997.

Levine MM, Galen J, Barry E, Noriega F, Tacket C, Sztein M, Chatfield S, Dougan G, Losonsky G, Kotloff K. Attenuated Salmonella Typhi and Shigella as live oral vaccines and as live vectors. Behring Inst Mitt 98:120-123, 1997.

Tacket CO, Kelly SM, Schödel F, Losonsky G, Nataro JP, Edelman R, Levine MM, Curtiss R III. Safety and immunogenicity in humans of an attenuated Salmonella Typhivaccine vector strain expressing plasmid-encoded hepatitis B antigens stabilized by the Asd-balanced lethal system. Infect Immun 65:3381-3385, 1997.

González CR, Noriega FR, Huerta S, Santiago A, Vega M, Paniagua J, Ortiz-Navarrete V, Isibasi A, Levine MM. Immunogenicity of a Salmonella Typhi CVD 908 candidate vaccine strain expressing the major surface protein gp63 of Leishmania mexicana mexicana. Vaccine 16:9/10 1043-1052, 1998.

Orr N, Galen JE, Levine MM. Expression and immunogenicity of a mutant diphtheria toxin molecule, CRM197, and its fragments in Salmonella Typhi vaccine strain CVD 908-htrA. Infect Immun 67:4290-4294, 1999.

Levine MM, Ferreccio C, Abrego P, San Martin O, Ortiz E, Cryz SC. Duration of efficacy of Ty21a, attenuated Salmonella Typhi live oral vaccine. Vaccine 17:2 Supplement S22-527, 1999.

Pasetti MF, Anderson RJ, Noriega FR, Levine MM, Sztein MB. Attenuated ΔguaBA Salmonella Typhi vaccine strain CVD 915 as a live vector utilizing prokaryotic or eukaryotic expression systems to deliver foreign antigens and elicit immune responses. Clin Immun 92:76-89, 1999.

Galen JE, Nair J, Wang JY, Tanner MK, Sztein MB, Levine MM. Optimization of plasmid maintenance in the attenuated live vector vaccine Salmonella Typhi strain CVD 908-htrA. Infect Immun 67:6424-6433, 1999.

Kotloff KL, Noriega FR, Samandari T, Sztein MB, Losonsky GA, Nataro JP, Picking WD, Levine MM. Shigella flexneri 2a strain CVD 1207 with specific deletions in virG, sen, set and guaBA is highly attenuated in humans. Infect Immun 68:1034-39, 2000.

Tacket CO, Sztein MB, Wasserman SS, Losonsky G, Kotloff KL, Wyant TL, Nataro JP, Edelman R, Perry J, Bedford P, Brown D, Chatfield S, Dougan G, Levine MM. Phase 2 clinical trial of attenuated Salmonella enterica serovar Typhi oral live vector vaccine CVD 908-htrA in U.S. volunteers. Infect Immun 68:1196-1201, 2000.

Anderson RJ, Pasetti MF, Sztein MB, Levine MM, Noriega FR. ΔguaBA attenuated Shigella flexneri 2a strain CVD 1204 as a Shigella vaccine and as a live mucosal delivery system for fragment C of tetanus toxin. Vaccine 18:2193-2202, 2000.

Tacket CO, Galen J, Sztein MB, Losonsky G, Wyant TL, Nataro J, Wasserman SS, Edelman R, Chatfield S, Dougan G, Levine MM. Safety and immune responses to attenuated Salmonella enterica serovar Typhi oral live vector vaccines expressing tetanus toxin fragment C. Clin Immunol 97:146-153, 2000.

Pasetti MF, Tanner MK, Pickett TE, Levine MM, Sztein M. Mechanisms and cellular events associated with the priming of mucosal and systemic immune responses to Salmonella enterica serovar Typhi live vector vaccines delivered intranasally in the murine model. Vaccine 18:3208-3213. 2000.

Wu S, Beier M, Sztein M, Galen JE, Pickett T, Holder AA, Gómez-Duarte O, Levine MM. Construction and immunogenicity in mice of attenuated Salmonella Typhi expressing Plasmodium falciparum merozoite surface protein (MSP-1) fused to tetanus toxin fragment C. J Biotechnol. 83:125-135, 2000.

Wang JY, Noriega FR, Galen JE, Barry E, Levine MM. Constitutive expression of the Vi polysaccharide capsular antigen in attenuated Salmonella enterica serovar Typhi oral vaccine strain CVD 909. Infect Immun 68:4647-4652, 2000.

Koprowski H II, Levine MM, Anderson RA, Losonsky G, Pizza M, Barry EM. Attenuated Shigella flexneri 2a vaccine strain CVD 1204 expressing colonization factor antigen I and mutant heat-labile enterotoxin of enterotoxigenic Escherichia coli. Infect Immun 68:4884-92, 2000.

Gómez-Duarte O, Pasetti M, Santiago A, Sztein MB, Hoffman SL, Levine MM. Expression, secretion and immunogenicity of the Plasmodium falciparum SSP-2 protein in Salmonella vaccine strains by a type I secretion system. Infect Immun 69:1192-1198, 2001.

Orr N, Galen JE, Levine MM. Novel use of anaerobically induced promoter, dmsA, for controlled expression of Fragment C of tetanus toxin in live attenuated Salmonella enterica serovar Typhi strain CVD 908-htrA. Vaccine 19:1694-1700, 2001.

Altboum Z, Barry EM, Losonsky G, Galen JE, Levine MM. Attenuated Shigella flexneri 2a ΔguaBA strain CVD 1204 expressing ETEC CS2 and CS3 fimbriae as a live mucosal vaccine against Shigella and enterotoxigenic Escherichia coli infection. Infect Immun 69:3150-8, 2001.

Wang JY, Pasetti MF, Noriega FR, Anderson RS, Wasserman SS, Galen JE, Sztein MB, Levine MM. Construction, genotypic and phenotypic characterization, and immunogenicity of attenuated ΔguaBA Salmonella enterica serovar Typhi strain CVD 915. Infect Immun 69:4734-4741, 2001.

Galen JE, Levine MM. Can a "flawless" live vector vaccine strain be engineered? Trends Microbiol 9:372-376, 2001.

Kotloff KL, Taylor DN, Sztein MB, Wasserman SS, Losonsky GA, Nataro JP, Venkatesan M, Hartman A, Picking WD, Katz DE, Campbell JD, Levine MM, Hale TL.. Phase I evaluation of ΔvirG Shigella sonnei live, attenuated, oral vaccine strain WRSS1 in healthy adults. Infect Immun 70:2016-21, 2002.

Pasetti, M, Levine MM, Sztein MB. Animal models paving the way for clinical trials of attenuated Salmonella enterica serovar Typhi live oral vaccines and live vectors. Vaccine. 21:401-18, 2003.

Pasetti MF, Barry EM, Losonsky G, Singh M, Medina-Moreno,SM, Polo JM, Robinson H, Sztein MB, Levine MM. Attenuated Salmonella enterica serovar Typhi and Shigella flexneri 2a strains mucosally deliver DNA vaccines encoding measles virus hemagglutinin, inducing specific immune responses and protection in cotton rats. J Virol 77:5209-5217, 2003.

Salerno-Goncalves R, Wyant TL, Pasetti MF, Fernandez-Vina M, Tacket CO, Levine MM, Sztein MB. Concomitant Induction of CD4(+) and CD8(+) T Cell Responses in Volunteers Immunized with Salmonella enterica Serovar Typhi Strain CVD 908-htrA. J Immol. 170:2734-2741,2003.

Tacket CO, Pasetti MF, Sztein, MB, Livio S, Levine MM. Immune responses to an oral Typhoid vaccine strain modified to constitutively express Vi capsular polysaccharide. J Infect Dis, 190:565-570, 2004.

Vindurampulle CJ, Cuberos LF, Barry EM, Pasetti MF, Levine MM. Recombinant Salmonella enterica serovar Typhi in a prime-boost strategy. Vaccine 22(27-28):3744-3750, 2004.

Capozzo AV, Cuberos L, Levine MM, Pasetti MF. Mucosally delivered Salmonella live vector vaccines elicit potent immune responses against a foreign antigen in neonatal mice born to naive and immune mothers. Infect Immun 72:4637-4646, 2004.

Kotloff KL, Pasetti MF, Barry EM, Nataro JP, Wasserman SS, Sztein MB, Picking WD, Levine MM. Deletion in the Shigella enterotoxin genes further attenuates Shigella flexneri 2a bearing guanine auxotrophy in a Phase 1 trial of CVD 1204 and CVD 1208. J Infect Dis 190:1745-1754, 2004.

Galen JE, Zhao L, Chinchilla M, Wang JY, Pasetti MF, Green J, Levine MM. Adaptation of the endogenous Salmonella enterica serovar Typhi clyA-encoded hemolysin for antigen export enhances the immunogenicity of anthrax protective antigen domain 4 expressed by the attenuated live vector vaccine strain CVD 908-htrA. Infect Immun 72:7096-7106,2004.

## Claims

1. A method for preparing a conjugate vaccine, comprising:
(a) mutating a strain of *Salmonella* Paratyphi A to generate an attenuated strain of *Salmonella* Paratyphi A, wherein said attenuated *Salmonella* Paratyphi A strain comprises a mutation of the *clpP* gene and one or more further genetic mutations selected from the group consisting of a mutation of the *guaBA* loci, a mutation of the *guaB* gene and a mutation of the *guaA* gene;
(b) isolating an O polysaccharide (OPS) and a flagellin protein from the attenuated strain of (a), wherein said flagellin protein is phase 1 flagellin (fliC); and
(c) conjugating the OPS to the flagellin protein, thereby preparing a conjugate vaccine.

2. The method of claim 1, wherein said OPS of *Salmonella* Paratyphi A belongs to Group A OPS of *Salmonella.*

3. The method of claim 1 or 2, wherein said attenuated *Salmonella* Paratyphi A strain further comprises one or more genetic mutations selected from the group consisting of a mutation of the *clpX* gene, a mutation of the *fliD* gene, a mutation of the *flgK* gene, and a mutation of the *flgL* gene.

4. The method of claim 1, wherein said attenuated *Salmonella* Paratyphi A strain comprises a mutation of the *guaBA* loci and a mutation of the *clpP* gene.

5. The method of claim 4, wherein the mutation of the *guaBA* loci and the mutation of the clpP gene is a deletion of part or all of the *guaBA* loci and a deletion of part or all of the *clpP* gene.

6. The method of claim 1, wherein the OPS and the flagellin protein are conjugated by chemical conjugation using a cross-linker or a polymer.

## Patentansprüche

1. Verfahren zur Herstellung eines Konjugatimpfstoffs, umfassend:
(a) Mutieren eines Stamms von *Salmonella* Paratyphi A zur Erzeugung eines abgeschwächten Stamms von *Salmonella* Paratyphi A, wobei der abgeschwächte Stamm von *Salmonella* Paratyphi A eine Mutation des *clpP*-Gens und ein oder mehr weitere genetische Mutationen umfasst, ausgewählt aus der Gruppe, bestehend aus einer Mutation der *guaBA*-Loci, einer Mutation des *guaB*-Gens und einer Mutation des *guaA*-Gens;
(b) Isolieren eines O-Polysaccharids (OPS) und eines Flagellinproteins aus dem abgeschwächten Stamm von (a), wobei das Flagellinprotein Phase 1-Flagellin (fliC) ist; und
(c) Konjugieren des OPS zu dem Flagellinprotein und dadurch Herstellen eines Konjugatimpfstoffs.

2. Verfahren nach Anspruch 1, wobei das OPS von *Salmonella* Paratyphi A zur Gruppe A OPS von *Salmonella* gehört.

3. Verfahren nach Anspruch 1 oder 2, wobei der abgeschwächte Stamm von *Salmonella* Paratyphi A weiter ein oder mehr genetische Mutationen umfasst, ausgewählt aus der Gruppe, bestehend aus einer Mutation des *clpX*-Gens, einer Mutation des *fliD*-Gens, einer Mutation des *flgK*-Gens und einer Mutation des *flgL*-Gens*.*

4. Verfahren nach Anspruch 1, wobei der geschwächte Stamm von *Salmonella* Paratyphi A eine Mutation der *guaBA*-Loci und eine Mutation des *clpP*-Gens umfasst.

5. Verfahren nach Anspruch 4, wobei die Mutation der *guaBA*-Loci und die Mutation des *clpP*-Gens eine teilweise oder vollständige Deletion der *guaeA*-Loci und eine teilweise oder vollständige Deletion des *clpP*-Gens ist.

6. Verfahren nach Anspruch 1, wobei das OPS und das Flagellinprotein durch chemische Konjugation unter Verwendung eines Crosslinkers oder eines Polymers konjugiert wird.

## Revendications

1. Procédé pour une préparation d'un vaccin conjugué, comprenant:
(a) muter une souche de *Salmonella* Paratyphi A pour générer une souche atténuée de *Salmonella* Paratyphi A; dans lequel ladite souche atténuée de *Salmonella* Paratyphi A comprend une mutation du gène *clpP,* et une ou plusieurs autres mutations génétiques choisies dans le groupe constitué par une mutation des loci *guaBA,* une mutation du gène *guaB* et une mutation du gène *guaA;*
(b) isoler un polysaccharide-O (PS-O) et une protéine flagelline de la souche atténuée de (a), dans lequel la protéine flagelline est la flagelline de phase 1 (*fliC*); et
(c) conjuguer le PS-O avec la protéine flagelline, préparant ainsi un vaccin conjugué.

2. Procédé selon la revendication 1, dans lequel ledit PS-O de *Salmonella* Paratyphi A appartient au groupe A du PS-O de *Salmonella.*

3. Procédé selon la revendication 1 ou 2, dans lequel ladite souche atténuée de *Salmonella* Paratyphi A comprend en outre une ou plusieurs mutations génétiques choisie(s) dans le groupe constitué par une mutation du gène *clpX,* une mutation du gène *fliD,* une mutation du gène *flgK* et une mutation du gène *flgL.*

4. Procédé selon la revendication 1, dans lequel ladite souche atténuée de *Salmonella* Paratyphi A comprend une mutation des loci *guaBA* et une mutation du gène *clpP.*

5. Procédé selon la revendication 4, dans lequel la mutation des loci de *guaBA* et une mutation du gène *clpP* est une délétion d'une partie ou de la totalité des loci *guaBA* et une délétion d'une partie ou de la totalité du gène *clpP.*

6. Procédé selon la revendication 1, dans lequel le PS-O et la protéine flagelline sont conjuguées par conjugaison chimique en utilisant un agent de réticulation ou un polymère.


### SEQUENCE LISTING

<110> VINDURAMPULLE, Christofer
   BARRY, Eileen
   LEVINE, Myron
   GALEN, James
<120> ATTENUATED SALMONELLA ENTERICA SEROVAR PARATYPHI A AND USES THEREOF
<130> 56995
<150> US 12/399,091
   <151> 2009-03-06
<160> 50
<170> PatentIn version 3.5
<210> 1
   <211> 489
   <212> DNA
   <213> Artificial Sequence
<220>
   <223> chemically-synthesized mutated guaBA region
<400> 1
   ggaacatcgc acagcgcacc tgagcggtat cgtctttgag cgtaaagtac cagtggcccg 60
   acgcaggctg cgtgaaatta gaaatctcgc cgctgatcca tacctgtccc atctcctgtt 120
   ctaacagcag acgaaccgtc tggttaaggc ggcttacggt aaaaattgag gaagtttgag 180
   aggataacat gtgagcggga tcaaattcta aatcagcagg ttattcaatc ggtgtaggct 240
   ggagcctgct tcgaagttcc tatactttct agagaatagg aacttcggaa taggaactaa 300
   ggaggatatt catatgctct ctctctgcgt gctgtcgaaa ccatcgactt tatgaccgcg 360
   cactgggcgc acctgccgta tgacttcctg ggtcgtgttt ccaaccgcat catcaatgaa 420
   gtcaacggga tttcccgtgt ggtgtatgac atcagcggta aaccaccggc taccattgag 480
   tgggaatga 489
<210> 2
   <211> 634
   <212> DNA
   <213> Artificial Sequence
<220>
   <223> chemically-synthesized mutated clpX region
<400> 2
   cctgagaatg gcatttgcgt cgtcgtgtgc ggcacaaaga acaaagaaga ggttttgact 60
   catgacagat aaacgcaaag atggctcggg caaattgttg tactgctctt tttgcggcaa 120
   aagccagcat gaagtgcgca agctgattgc cggtccatcc gtgtatatct gcgacgaatg 180
   cgtcgattta tgtaacgaca ttattcgcgc ccttatatat atgcggccgc tgtaggctgg 240
   agctgcttcg aagttcctat actttctaga gaataggaac ttcggaatag gaactaagga 300
   ggatattcat atggcgcgcc tataagggcg aattccgtga cgaagcgctg aacgctatcg 360
   ccaggaaagc aatggcgcgt aaaaccggtg cccgtggtct gcgttctatc gtcgaagcgg 420
   cgctgctgga taccatgtac gatttgccat ctatggaaga cgtcgaaaaa gtggtgatcg 480
   acgagtccgt tattgccggt cagagtaagc cgttgctgat ttacggcaaa ccggaagcgc 540
   aggcttctgg cgaataatta aacattcata caatcagtta gccaaaaaag gggggatttt 600
   atctcccctt tcgtttttcc tgtaaacacg ccgt 634
<210> 3
   <211> 370
   <212> DNA
   <213> Artificial Sequence
<220>
   <223> chemically-synthesized mutated clpP region
<400> 3
   tccatcaggt tacaatcagt acagcaggtt ttttcaattt ttatccagga gacggaaatg 60
   tcatacagcg gagaacgaga taatttggcc cctcatatat gaatatcctc cttagttcct 120
   attccgaagt tcctattctc tagaaagtat aggaacttcg aagcagctcc agcctacacg 180
   ctaggactca attttgaccc atcgtaattg atgccctgga cgcaagtgtg ccgctataca 240
   cttcatcctt cacgctacct cggtgttggt tgccagcgcg cctcccggtg acttacttat 300
   gtaagcgcct gcggagtcgc cgagttgccg ccttgatgta gctcgaatga ttttgtgtat 360
   atactaatga 370
<210> 4
   <211> 27
   <212> DNA
   <213> Artificial Sequence
<220>
   <223> chemically-synthesized oligonucleotide primer
<400> 4
   ctgcagtcat tcccactcaa tggtagc 27
<210> 5
   <211> 18
   <212> DNA
   <213> Artificial Sequence
<220>
   <223> chemically-synthesized oligonucleotide primer
<400> 5
   ggaacatcgc acagcgca 18
<210> 6
   <211> 17
   <212> DNA
   <213> Artificial Sequence
<220>
   <223> chemically-synthesized oligonucleotide primer
<400> 6
   gtgtaggagc tgcttcg 17
<210> 7
   <211> 20
   <212> DNA
   <213> Artificial Sequence
<220>
   <223> chemically-synthesized oligonucleotide primer
<400> 7
   catatgaata tcctccttag 20
<210> 8
   <211> 119
   <212> DNA
   <213> Artificial Sequence
<220>
   <223> chemically-synthesized oligonucleotide primer
<400> 8
   cgaaccgtct ggttaaggcg gcttacggta aaaattgagg aagtttgaga ggataacatg 60
   tgagcgggat caaattctaa atcagcaggt tattcaatcg tgtaggctgg agctgcttc 119
<210> 9
   <211> 120
   <212> DNA
   <213> Artificial Sequence
<220>
   <223> chemically-synthesized oligonucleotide primer
<400> 9
   ttcattgatg atgcggttgg aaacacgacc caggaagtca tacggcaggt gcgcccagtg 60
   cgcggtcata aagtcgatgg tttcgacagc acgcagagag catatgaata tcctccttag 120
<210> 10
   <211> 28
   <212> DNA
   <213> Artificial Sequence
<220>
   <223> chemically-synthesized oligonucleotide primer
<400> 10
   gaaggagtat tgcccatgct acgtatcg 28
<210> 11
   <211> 42
   <212> DNA
   <213> Artificial Sequence
<220>
   <223> chemically-synthesized oligonucleotide primer
<400> 11
   catatgaagg agtattgccc atgctacgta tcgctaaaga ag 42
<210> 12
   <211> 36
   <212> DNA
   <213> Artificial Sequence
<220>
   <223> chemically-synthesized oligonucleotide primer
<400> 12
   atgcatctgc agtcattccc actcaatggt agccgg 36
<210> 13
   <211> 30
   <212> DNA
   <213> Artificial Sequence
<220>
   <223> chemically-synthesized oligonucleotide primer
<400> 13
   acagataaac gcaaagatgg ctcgggcaaa 30
<210> 14
   <211> 30
   <212> DNA
   <213> Artificial Sequence
<220>
   <223> chemically-synthesized oligonucleotide primer
<400> 14
   ttattcgcca gaagcctgcg cttccggttt 30
<210> 15
   <211> 30
   <212> DNA
   <213> Artificial Sequence
<220>
   <223> chemically-synthesized oligonucleotide primer
<400> 15
   cctgagaatg gcatttgcgt cgtcgtgtgc 30
<210> 16
   <211> 30
   <212> DNA
   <213> Artificial Sequence
<220>
   <223> chemically-synthesized oligonucleotide primer
<400> 16
   acggcgtgtt tacaggaaaa acgaaagggg 30
<210> 17
   <211> 30
   <212> DNA
   <213> Artificial Sequence
<220>
   <223> chemically-synthesized oligonucleotide primer
<400> 17
   tcatacagcg gagaacgaga taatttggcc 30
<210> 18
   <211> 30
   <212> DNA
   <213> Artificial Sequence
<220>
   <223> chemically-synthesized oligonucleotide primer
<400> 18
   ttacataagt aagtcactgg gaggcgcgct 30
<210> 19
   <211> 30
   <212> DNA
   <213> Artificial Sequence
<220>
   <223> chemically-synthesized oligonucleotide primer
<400> 19
   tccatcaggt tacaatcagt acagcagatt 30
<210> 20
   <211> 30
   <212> DNA
   <213> Artificial Sequence
<220>
   <223> chemically-synthesized oligonucleotide primer
<400> 20
   tcattagtat atacacaaaa tcattcgagc 30
<210> 21
   <211> 45
   <212> DNA
   <213> Artificial Sequence
<220>
   <223> chemically-synthesized oligonucleotide primer
<400> 21
   gcggccgcga aggagagacg gaaatgtcat acagcggaga acgag 45
<210> 22
   <211> 42
   <212> DNA
   <213> Artificial Sequence
<220>
   <223> chemically-synthesized oligonucleotide primer
<400> 22
   tcgcgagaat tcttacataa gtaagtcact gggaggcgcg ct 42
<210> 23
   <211> 45
   <212> DNA
   <213> Artificial Sequence
<220>
   <223> chemically-synthesized oligonucleotide primer
<400> 23
   gcggccgcga aggagtttga ctcatgacag ataaacgcaa agatg 45
<210> 24
   <211> 36
   <212> DNA
   <213> Artificial Sequence
<220>
   <223> chemically-synthesized oligonucleotide primer
<400> 24
   catatgttat tcgccagaag cctgcgcttc cggttt 36
<210> 25
   <211> 38
   <212> DNA
   <213> Artificial Sequence
<220>
   <223> chemically-synthesized oligonucleotide primer
<400> 25
   gcggccgctt acataagtaa gtcactggga ggcgcgct 38
<210> 26
   <211> 3120
   <212> DNA
   <213> Salmonella Typhi Ty2
<400> 26
   ttgcccatgc tacgtatcgc taaagaagcc ctgacgtttg acgacgtcct ccttgttccc 60
   gctcactcca ccgttttgcc gaatactgct gatctcagca cgcagttgac gaaaactatt 120
   cgtctgaata ttcctatgct ttctgcggcg atggacaccg tgacggaagc gcgcctggca 180
   attgccctgg cccaggaagg cggcatcggt tttatccaca aaaacatgtc tattgagcgc 240
   caggcggaag aagttcgccg cgtgaagaaa cacgagtccg gcgtagtgac cgacccgcag 300
   accgtcctgc caaccaccac gttgcatgaa gtgaaagccc tgaccgagcg taacggtttt 360
   gcgggctatc cggtggtgac tgaagataac gagctggtgg gtatcatcac cggtcgtgac 420
   gtgcgttttg tgactgacct gaaccagccg gtgagtgttt acatgacgcc gaaagagcgt 480
   ctggtgaccg ttcgtgaagg cgaagcccgt gaagtcgtgc tggcaaaaat gcacgaaaaa 540
   cgcgtagaaa aagcgctggt cgttgatgat aacttccatc tgcttggcat gattaccgta 600
   aaagatttcc agaaagcgga acgtaaacca aactcctgta aagatgagca gggccgttta 660
   cgtgtcggcg cggcggtcgg cgcaggcgcg ggcaacgaag agcgcgttga cgcgctggtg 720
   gcggcaggcg ttgacgtcct gctgatcgac tcttctcacg gtcactctga aggcgtgttg 780
   caacgtatcc gtgaaacccg tgctaaatat cctgacctgc aaatcatcgg cggcaacgtc 840
   gcgacgggcg caggcgctcg cgcactggcg gaagccggtt gcagcgcggt gaaagtcggt 900
   atcggcccgg gttccatctg taccactcgt atcgtgactg gcgtgggcgt tccgcagatt 960
   accgctgttt ctgacgcagt tgaagcgctg gaaggcaccg ggattccggt tatcgctgac 1020
   ggcggtatcc gtttctccgg cgacatcgcc aaagccatcg ccgcaggcgc gagcgctgtc 1080
   atggtcggtt ctatgctggc gggtaccgaa gaatccccgg gcgaaatcga actctaccag 1140
   ggccgttctt acaaatctta ccgcggcatg ggctcgctgg gcgcgatgtc caaaggttcc 1200
   tctgaccgtt acttccagag cgacaacgcc gccgacaaac tggtgccgga aggtatcgaa 1260
   ggccgcgtag cctataaagg tcgcctgaaa gagatcattc accagcagat gggcggcctg 1320
   cgctcctgta tggggctgac cggttgtgct accatcgacg aactgcgtac taaagcggag 1380
   tttgtgcgta tcagcggtgc gggtatccag gaaagccacg ttcacgacgt gaccatcacc 1440
   aaagagtccc cgaactaccg tctgggctcc tgattttctt cgcccgacct tcgcgtcggg 1500
   cgatttattt aatctgtttc acttgcctcg gaataagcgt caatgacgga aaacattcat 1560
   aagcatcgca tcctcattct ggacttcggt tctcagtaca ctcaactggt tgcgcgccgc 1620
   gtgcgtgagc tgggtgttta ctgcgaactg tgggcgtggg atgtgacaga agcacaaatt 1680
   cgtgacttca acccaagcgg cattattctt tccggcggcc cggaaagcac caccgaagaa 1740
   aacagcccgc gcgcgccgca gtatgtcttt gaagcaggcg tgccggtatt tggcgtctgc 1800
   tacgggatgc agaccatggc gatgcagctt ggcggtcatg tagaaggttc taatgagcgt 1860
   gaatttggtt acgcgcaggt cgaagtgctg accgacagcg cgctggttcg cggtattgaa 1920
   gattccctga ccgccgacgg caaaccgctg ctggacgtgt ggatgagcca cggcgataaa 1980
   gtgacggcga ttccgtccga cttcgtgacc gtagccagca ccgaaagctg cccgttcgcc 2040
   atcatggcta acgaagaaaa acgcttctac ggcgtacagt tccacccgga agtgactcac 2100
   acccgccagg gtatgcgcat gctggagcgt tttgtgcgtg atatctgcca gtgtgaagcc 2160
   ctgtggacgc cggcgaagat catcgacgac gccgtggcgc gcattcgcga gcaggtaggc 2220
   gacgataaag tgatcctcgg tctctccggc ggcgtggatt cttccgtaac cgcaatgctg 2280
   ctgcaccgcg cgatcggtaa aaatctgacc tgtgtattcg tcgacaacgg cctgctgcgt 2340
   ctcaacgaag ccgagcaggt gatggacatg tttggcgacc attttggtct gaacatcgtt      2400
   cacgtaccgg cagaagatcg cttcctgtcc gcgttggctg gcgaaaacga tccggaagcg      2460
   aagcgtaaga tcattggccg tgtttttgtg gaagtgttcg acgaagaagc gttgaaactg      2520
   gaagacgtga aatggctggc gcagggcacc atctaccctg acgtcatcga atctgcggcg      2580
   tctgcaaccg gtaaagcgca cgtcatcaaa tctcaccaca atgttggcgg cctgccgaaa      2640
   gagatgaaga tggggctggt tgaaccgctg aaagagctgt tcaaagacga agtgcgtaag      2700
   attggtctgg agctgggcct gccgtacgac atgctgtacc gtcatccgtt cccggggccg      2760
   ggcctcggcg tacgtgtact gggtgaagtg aagaaagagt actgcgacct gctgcgccgt      2820
   gctgatgcca tcttcattga agagctgcgt aaggcggatc tgtacgacaa agtcagccag      2880
   gcgttcaccg tcttcctgcc agtacgctcc gttggcgtaa tgggcgatgg tcgtaagtac      2940
   gattgggtgg tctctctgcg tgctgtcgaa accatcgact ttatgaccgc gcactgggcg      3000
   cacctgccgt atgacttcct gggtcgtgtt tccaaccgca tcatcaatga agtcaacggg      3060
   atttcccgtg tggtgtatga catcagcggt aaaccaccgg ctaccattga gtgggaataa      3120
<210> 27
   <211> 2147
   <212> DNA
   <213> Salmonella Typhi Ty2
<400> 27
   ttattcgcca gaagcctgcg cttccggttt gccgtaaatc agcaacggct tactctgacc      60
   ggcaataacg gactcgtcaa tcaccacttt ttcgacgtct tccatagatg gcaaatcgta     120
   catggtatcc agcagcgccg cttcgacgat agaacgcagg ccacgggcac cagttttacg     180
   cgccatcgct ttcctggcga tagcgttcag cgcttcgtca cggaattcca gatcgacgcc     240
   ttccaggtta aacagcgcct gatactgctt ggtcagcgca tttttcggct ctttcaggat     300
   ttgaaccagc gcttcttcgc tgagttcgtt cagcgtcgcc accactggca gacgaccgat     360
   aaactcagga atcagaccaa atttgatcaa atcttccggt tcaacctgcg acaacagctc     420
   gccttcactg gctttgtcgg acttcgcttt caccgtcgcg ccaaaaccaa tgccggagcc     480
   ggtttcaaca cggttagcga tcactttatc cagaccggca aacgcgccgc cgcagataaa     540
   cagaatctta gaggtatcta cctgtaagaa ctcctgctgc ggatgtttgc gaccgccctg     600
   cggtggaacc gcggcgacgg tgccttcgat cagtttcagc aacgcctgct gtacgccttc     660
   gccggaaaca tcgcgggtaa tggacggatt gtctgattta cgcgaaatct tatcgatttc     720
   atcaatgtag acaatcccac gctgcgcttt ttgcacgtcg tagtcgcatt tctgcaacag     780
   tttctgaatg atattctcga cgtcttcccc cacgtaaccc gcttcggtca gcgtggtcgc     840
   atccgccata gtgaacggca catccagcaa gcgcgccagc gtttccgcca gcagcgtttt     900
   accggaaccg gtcggtccag tcagcagaat gttgcttttg cctaactcga cgccattgct     960
   ggtatcgccg ttacgcagac gcttgtagtg gttatagacc gccaccgcca gcactttttt 1020
   cgcctgctcc tggccgataa cgtaatcgtc caggtgagta cgaatttcat gcggcgtcgg 1080
   cagcgcacta cgttcacggt gcggagcaac ttctttaatt tcttcgcgaa taatgtcgtt 1140
   acataaatcg acgcattcgt cgcagatata cacggatgga ccggcaatca gcttgcgcac 1200
   ttcatgctgg cttttgccgc aaaaagagca gtacaacaat ttgcccgagc catctttgcg 1260
   tttatctgtc atgagtcaaa acctcttctt tgttctttgt gccgcacacg acgacgcaaa 1320
   tgccattctc aggcgcaagc cgctaatcag cgttgtgccg cccttcatta gtatatacac 1380
   aaaatcattc gagctacatc aaggcggcaa ctcagcgact ccgcaggcgc ttacataagt 1440
   aagtcactgg gaggcgcgct ggcaaccaac accgaggtag cgtgaaggat gaagtgtata 1500
   gcggcacact tgcgtccagg gcatcaatta cgatgggtca aaattgagtc aaccaaaccg 1560
   tactctaccg cttcaggcgc ggagaggaag cgatcgcgct cagtatcacg ttcaatctgc 1620
   tcaagagatt gacccgtatg atgcgccata agttcattca tgcgcccttt tactttcaaa 1680
   atttcgcggg cgtgaatttc aatatccgtc gcctggccct gatagccgcc cagcggctgg 1740
   tggatcatga cgcgagagtt cggcaagcag aaacgtttgc ctttcgcccc ggcagtcagc 1800
   agaaacgccc ccatagaggc cgcctgtccc atacaaatgg tgctgacgtc tggcttaata 1860
   aactgcatgg tgtcatagat ggacatcccc gcagtaatta cgccgccagg agaattaatg 1920
   tacagataga tatctttttc cgggttttcc gcttccagga acagcatctg cgccacgatc 1980
   aggttagcca tatggtcttc gacctggccg gtcagaaata tgacgcgttc cttaagtaga 2040
   cgagaataga tatcaaaaga gcgctcaccg cgtgaggtct gttcaatgac catcggcacc 2100
   agcgccatat gaggggccaa attatctcgt tctccgctgt atgacat 2147
<210> 28
   <211> 687
   <212> DNA
   <213> E. coli
<400> 28
   cctagggcca gcaaaaggcc aggaaccgta aaaaggccgc gttgctggcg tttttccata 60
   ggctccgccc ccctgacgag catcacaaaa atcgacgctc aagtcagagg tggcgaaacc 120
   cgacaggact ataaagatac caggcgtttc cccctggaag ctccctcgtg cgctctcctg 180
   ttccgaccct gccgcttacc ggatacctgt ccgcctttct cccttcggga agcgtggcgc 240
   tttctcatag ctcacgctgt aggtatctca gttcggtgta ggtcgttcgc tccaagctgg 300
   gctgtgtgca cgaacccccc gttcagcccg accgctgcgc cttatccggt aactatcgtc 360
   ttgagtccaa cccggtaaga cacgacttat cgccactggc agcagccact ggtaacagga 420
   ttagcagagc gaggtatgta ggcggtgcta cagagttctt gaagtggtgg cctaactacg 480
   gctacactag aaggacagta tttggtatct gcgctctgct gaagccagtt accttcggaa 540
   aaagagttgg tagctcttga tccggcaaac aaaccaccgc tggtagcggt ggtttttttg 600
   tttgcaagca gcagattacg cgcagaaaaa aaggatctca agaagatcct ttgatctttt 660
   ctacggggtc tgacgctcag tagatct 687
<210> 29
   <211> 635
   <212> DNA
   <213> Salmonella typhimurium
<400> 29
   cctaggagat acttaacagg gaagtgagag ggccgcggca aagccgtttt tccataggct 60
   ccgcccccct gacaagcatc acgaaatctg acgctcaaat cagtggtggc gaaacccgac 120
   aggactataa agataccagg cgtttccccc tggcggctcc ctcgtgcgct ctcctgttcc 180
   tgcctttcgg tttaccggtg tcattccgct gttatggccg cgtttgtctc attccacgcc 240
   tgacactcag ttccgggtag gcagttcgct ccaagctgga ctgtatgcac gaaccccccg 300
   ttcagtccga ccgctgcgcc ttatccggta actatcgtct tgagtccaac ccggaaagac 360
   atgcaaaagc accactggca gcagccactg gtaattgatt tagaggagtt agtcttgaag 420
   tcatgcgccg gttaaggcta aactgaaagg acaagttttg gtgactgcgc tcctccaagc 480
   cagttacctc ggttcaaaga gttggtagct cagagaacct tcgaaaaacc gccctgcaag 540
   gcggtttttt cgttttcaga gcaagagatt acgcgcagac caaaacgatc tcaagaagat 600
   catcttatta atcagataaa atatttctag gatct 635
<210> 30
   <211> 1955
   <212> DNA
   <213> E. coli
<400> 30
   cctaggtttc acctgttcta ttaggtgtta catgctgttc atctgttaca ttgtcgatct      60
   gttcatggtg aacagcttta aatgcaccaa aaactcgtaa aagctctgat gtatctatct     120
   tttttacacc gttttcatct gtgcatatgg acagttttcc ctttgatatc taacggtgaa     180
   cagttgttct acttttgttt gttagtcttg atgcttcact gatagataca agagccataa     240
   gaacctcaga tccttccgta tttagccagt atgttctcta gtgtggttcg ttgtttttgc     300
   gtgagccatg agaacgaacc attgagatca tgcttacttt gcatgtcact caaaaatttt     360
   gcctcaaaac tggtgagctg aatttttgca gttaaagcat cgtgtagtgt ttttcttagt     420
   ccgttacgta ggtaggaatc tgatgtaatg gttgttggta ttttgtcacc attcattttt     480
   atctggttgt tctcaagttc ggttacgaga tccatttgtc tatctagttc aacttggaaa     540
   atcaacgtat cagtcgggcg gcctcgctta tcaaccacca atttcatatt gctgtaagtg     600
   tttaaatctt tacttattgg tttcaaaacc cattggttaa gccttttaaa ctcatggtag     660
   ttattttcaa gcattaacat gaacttaaat tcatcaaggc taatctctat atttgccttg     720
   tgagttttct tttgtgttag ttcttttaat aaccactcat aaatcctcat agagtatttg      780
   ttttcaaaag acttaacatg ttccagatta tattttatga atttttttaa ctggaaaaga      840
   taaggcaata tctcttcact aaaaactaat tctaattttt cgcttgagaa cttggcatag      900
   tttgtccact ggaaaatctc aaagccttta accaaaggat tcctgatttc cacagttctc      960
   gtcatcagct ctctggttgc tttagctaat acaccataag cattttccct actgatgttc     1020
   atcatctgag cgtattggtt ataagtgaac gataccgtcc gttctttcct tgtagggttt     1080
   tcaatcgtgg ggttgagtag tgccacacag cataaaatta gcttggtttc atgctccgtt     1140
   aagtcatagc gactaatcgc tagttcattt gctttgaaaa caactaattc agacatacat     1200
   ctcaattggt ctaggtgatt ttaatcacta taccaattga gatgggctag tcaatgataa     1260
   ttactagtcc ttttcctttg agttgtgggt atctgtaaat tctgctagac ctttgctgga     1320
   aaacttgtaa attctgctag accctctgta aattccgcta gacctttgtg tgtttttttt     1380
   gtttatattc aagtggttat aatttataga ataaagaaag aataaaaaaa gataaaaaga     1440
   atagatccca gccctgtgta taactcacta ctttagtcag ttccgcagta ttacaaaagg     1500
   atgtcgcaaa cgctgtttgc tcctctacaa aacagacctt aaaaccctaa aggcttaagt     1560
   agcaccctcg caagctcggg caaatcgctg aatattcctt ttgtctccga ccatcaggca     1620
   cctgagtcgc tgtctttttc gtgacattca gttcgctgcg ctcacggctc tggcagtgaa     1680
   tgggggtaaa tggcactaca ggcgcctttt atggattcat gcaaggaaac tacccataat     1740
   acaagaaaag cccgtcacgg gcttctcagg gcgttttatg gcgggtctgc tatgtggtgc     1800
   tatctgactt tttgctgttc agcagttcct gccctctgat tttccagtct gaccacttcg     1860
   gattatcccg tgacaggtca ttcagactgg ctaatgcacc cagtaaggca gcggtatcat     1920
   caacaggctt acccgtctta ctgtcaaccg gatct                                1955
<210> 31
   <211> 1686
   <212> DNA
   <213> Escherichia coli
<400> 31
   cacttttgtt acccgccaaa caaaacccaa aaacaaccca tacccaaccc aataaaacac 60
   caaaacaaga caaataatca ttgattgatg gttgaaatgg ggtaaacttg acaaacaaac 120
   ccacttaaaa cccaaaacat acccaaacac acaccaaaaa aacaccataa ggagttttat 180
   aaatgttggt attcattgat gacggttcaa caaacatcaa actacagtgg caggaaagcg 240
   acggaacaat taaacagcac attagcccga acagcttcaa acgcgagtgg gcagtctctt 300
   ttggtgataa aaaggtcttt aactacacac tgaacggcga acagtattca tttgatccaa 360
   tcagcccgga tgctgtagtc acaaccaata tcgcatggca atacagcgac gttaatgtcg 420
   ttgcagtgca tcacgcctta ctgaccagtg gtctgccggt aagcgaagtg gatattgttt 480
   gcacacttcc tctgacagag tattacgaca gaaataacca acccaatacg gaaaatattg 540
   agcgtaagaa agcaaacttc cggaaaaaaa ttacattaaa tggcggggat acattcacaa 600
   taaaagatgt aaaagtcatg cctgaatcta taccggcagg ttatgaagtt ctacaagaac 660
   tggatgagtt agattcttta ttaattatag atctcggggg caccacatta gatatttctc 720
   aggtaatggg gaaattatcg gggatcagta aaatatacgg agactcatct cttggtgtct 780
   ctctggttac atctgcagta aaagatgccc tttctcttgc gagaacaaaa ggaagtagct 840
   atcttgctga cgatataatc attcacagaa aagataataa ctatctgaag caacgaatta 900
   atgatgagaa caaaatatca atagtcaccg aagcaatgaa tgaagcactt cgtaaacttg 960
   agcaacgtgt attaaatacg ctcaatgaat tttctggtta tactcatgtt atggttatag 1020
   gcggtggcgc agaattaata tgcgatgcag taaaaaaaca cacacagatt cgtgatgaac 1080
   gttttttcaa aaccaataac tctcaatatg atttagttaa cggtatgtat ctcataggta 1140
   attaatgatg gacaagcgca gaaccattgc cttcaaacta aatccagatg taaatcaaac 1200
   agataaaatt gtttgtgata cactggacag tatcccgcaa ggggaacgaa gccgccttaa 1260
   ccgggccgca ctgacggcag gtctggcctt atacagacaa gatccccgga cccctttcct 1320
   tttatgtgag ctgctgacga aagaaaccac attttcagat atcgtgaata tattgagatc 1380
   gctatttcca aaagagatgg ccgattttaa ttcttcaata gtcactcaat cctcttcaca 1440
   acaagagcaa aaaagtgatg aagagaccaa aaaaaatgcg atgaagctaa taaattaatt 1500
   caattattat tgagttccct ttatccacta tcaggctgga taaagggaac tcaatcaagt 1560
   tattttctta ccagtcatta cataatcgtt attatgaaat aatcgtttgc actgtctctg 1620
   ttattcaggc aatttcaata aaggcacttg ctcacgctct gtcattttct gaaactcttc 1680
   atgctg 1686
<210> 32
   <211> 367
   <212> DNA
   <213> Escherichia coli
<400> 32
   gacagtaaga cgggtaagcc tgttgatgat accgctgcct tactgggtgc attagccagt      60
   ctgaatgacc tgtcacggga taatccgaag tggtcagact ggaaaatcag agggcaggaa     120
   ctgctgaaca gcaaaaagtc agatagcacc acatagcaga cccgccataa aacgccctga     180
   gaagcccgtg acgggctttt cttgtattat gggtagtttc cttgcatgaa tccataaaag     240
   gcgcctgtag tgccatttac ccccattcac tgccagagcc gtgagcgcag cgaactgaat     300
   gtcacgaaaa agacagcgac tcaggtgcct gatggtcgga gacaaaagga atattcagcg     360
   atttgcc                                                               367
<210> 33
   <211> 459
   <212> DNA
   <213> Escherichia coli
<400> 33
   ttctgtggta gcacagaata atgaaaagtg tgtaaagaag ggtaaaaaaa accgaatgcg      60
   aggcatccgg ttgaaatagg ggtaaacaga cattcagaaa tgaatgacgg taataaataa     120
   agttaatgat gatagcggga gttattctag ttgcgagtga aggttttgtt ttgacattca     180
   gtgctgtcaa atacttaaga ataagttatt gattttaacc ttgaattatt attgcttgat     240
   gttaggtgct tatttcgcca ttccgcaata atcttaaaaa gttcccttgc atttacattt     300
   tgaaacatct atagcgataa atgaaacatc ttaaaagttt tagtatcata ttcgtgttgg     360
   attattctgc atttttgggg agaatggact tgccgactga ttaatgaggg ttaatcagta     420
   tgcagtggca taaaaaagca aataaaggca tataacaga                            459
<210> 34
   <211> 734
   <212> DNA
   <213> Shigella flexneri 2a strain CVD 1208s
<400> 34
   catatgattg acctgaatga atatacagta ttggaatgca ttatccggag tgttgtgtaa     60
   caatgtctgg ccaggtttgt ttcccggaac cgaggtcaca acatagtaaa agcgctattg    120
   gtaatggtac aatcgcgcgt ttacacttat tcagaacgac aggagacacg aacatggcca    180
   gcagaggcgt aaacaaggtt attctcgttg gtaatctggg tcaggacccg gaagtacgct    240
   acatgccaaa tggtggcgca gttgccaaca ttacgctggc tacttccgaa tcctggcgtg    300
   ataaagcgac cggcgagatg aaagaacaga ctgaatggca ccgcgttgtg ctgttcggca    360
   aactggcaga agtggcgagc gaatatctgc gtaaaggttc tcaggtttat atcgaaggtc    420
   agctgcgtac ccgtaaatgg accgatcaat ccggtcagga tcgctacacc acagaagtcg    480
   tggtgaacgt tggcggcacc atgcagatgc tgggtggtcg tcagggtggt ggcgctccgg    540
   caggtggcaa tatcggtggt ggtcagccgc agggcggttg gggtcagcct cagcagccgc    600
   agggtggcaa tcagttcagc ggcggcgcgc agtctcgccc gcagcagtcc gctccggcag    660
   cgccgtctaa cgagccgccg atggactttg atgatgacat tccgttctga tttgtcatta    720
   aaacaatagc tagc                                                      734
<210> 35
   <211> 1467
   <212> DNA
   <213> Salmonella Paratyphi A 9150
<220>
   <221> misc_feature
   <223> guaB
<400> 35
   atgctacgta tcgctaaaga agccctgacg tttgacgacg tcctccttgt tcccgctcac        60
   tccaccgttt tgccgaatac tgccgatctc agcacgcagt tgacgaaaac tattcgtctg       120
   aatattccta tgctttctgc ggcgatggac accgtgacgg aagcgcgcct ggcaattgcc       180
   ctggcccagg aaggcggcat tggttttatc cacaaaaaca tgtccattga gcgccaggcg       240
   gaagaagttc gccgcgtgaa gaaacacgag tccggcgtag tgaccgaccc gcagaccgtc       300
   ctgccaacca ccacgttgca tgaagtgaaa gccctgaccg agcgtaacgg ttttgcgggc       360
   tatccggtgg tgactgaaga taacgagctg gtggggatca tcaccggtcg tgacgtgcgt       420
   tttgtgactg acctgaacca gccggtaagt gtctacatga cgccgaaaga gcgtctggtg       480
   accgttcgtg aaggcgaagc ccgtgaagtc gtgctggcaa aaatgcacga aaaacgcgta       540
   gaaaaagcgc tggtcgttga tgataacttc catctgcttg gcatgattac cgtaaaagat       600
   ttccagaaag cggaacgtaa accaaactcc tgtaaagatg agcagggccg tttacgtgtc       660
   ggcgcggcgg tcggcgcagg cgcgggcaac gaagagcgcg ttgacgcgct ggtggcggca       720
   ggcgttgacg tactgctgat cgactcctct cacggtcact ctgaaggcgt gttgcaacgt       780
   atccgtgaga cgcgtgctaa atatcctgac ctgcaaatca tcggcggcaa cgttgcgacg       840
   ggcgcaggcg ctcgcgcact ggcggaagcc ggttgcagcg cggtgaaagt gggtatcggc       900
   ccgggctcca tctgtaccac tcgtatcgtg actggtgtgg gcgttccgca gatcaccgct       960
   gtttccgacg cggtagaagc gctggaaggc accggaattc cggttatcgc tgacggcggt      1020
   atccgtttct ccggcgacat cgccaaagcc atcgccgcag gcgcgagcgc cgtgatggtg      1080
   ggctctatgc tggccggtac cgaagaatcc ccgggcgaaa tcgaactcta ccagggccgt      1140
   tcgtacaaat cttaccgcgg catgggctcg ctgggcgcga tgtccaaagg ttcctccgac      1200
   cgttacttcc agagcgacaa cgccgctgac aaactggtgc cggaaggtat cgaaggccgc      1260
   gtagcctata aaggtcgcct gaaagagatc attcaccagc agatgggcgg cctgcgctcc      1320
   tgtatggggc tgaccggttg tgctaccatc gacgaactgc gtactaaagc ggagtttgtg      1380
   cgtatcagcg gtgcgggtat ccaggaaagc cacgttcacg acgtgaccat caccaaagag      1440
   tccccgaact accgtctggg ctcctga                                          1467
<210> 36
   <211> 1578
   <212> DNA
   <213> Salmonella Paratyphi A 9150
<220>
   <221> misc_feature
   <223> guaA
<400> 36
   atgacggaaa acattcataa gcatcgcatc ctcattctgg acttcggttc tcagtacact      60
   caactggttg cgcgccgcgt gcgtgagctg ggtgtttact gcgaactgtg ggcgtgggat     120
   gtgacagaag cacaaattcg tgacttcaac ccaagcggca ttattctttc cggcggcccg     180
   gaaagcacca ccgaagaaaa cagcccgcgc gcgccgcagt atgtctttga agcaggcgtg     240
   ccggtatttg gcgtttgcta tggtatgcag accatggcga tgcagcttgg cggtcatgta     300
   gaaggttcta atgagcgtga atttggttat gcgcaggtcg aagtgttgac cgacagcgcg     360
   ctggttcgcg gtattgaaga ttccctgacc gcagacggca aaccgctgct ggacgtgtgg     420
   atgagccacg gcgataaagt gacggcgatt ccgtccgact tcgtgaccgt cgccagcacc     480
   gagagctgcc cgttcgccat catggctaac gaagaaaaac gcttctacgg cgtacagttc     540
   cacccggaag tgacccacac ccgccagggg atgcgcatgc tggagcgttt tgtgcgtgat     600
   atctgccagt gtgaagcgtt gtggacgccg gcgaagatca tcgacgacgc cgtggcgcgc     660
   attcgcgagc aggtaggcga cgataaagtg atcctcggtc tctccggcgg cgtggattct     720
   tccgtcaccg caatgctgct gcaccgcgcg atcggtaaaa atctgacctg tgtattcgtc     780
   gacaacggcc tgctgcgtct caacgaagcc gagcaggtga tggacatgtt tggcgaccat     840
   tttggcctga atatcgttca cgttccggcg gaagagcgct tcctgtccgc gttggctggc     900
   gaaaacgatc cggaagcgaa gcgtaagatc attggccgtg tttttgtgga agtgttcgac     960
   gaagaagcgt tgaaactgga agacgtgaaa tggctggcgc agggcaccat ctaccctgac    1020
   gtcatcgagt ctgcggcgtc tgcaaccggt aaagcgcacg tcatcaaatc tcaccacaat    1080
   gttggcggcc tgccgaaaga gatgaagatg gggctggttg aaccgctgaa agagctgttc    1140
   aaagacgaag tgcgtaagat tggtctggag ctgggcctgc cgtacgacat gctgtaccgt    1200
   catccgttcc cggggccggg cctcggcgta cgtgtactgg gtgaagtgaa gaaagagtac    1260
   tgcgacctgt tgcgccgtgc tgacgccatc ttcattgaag agctgcgtaa ggcggatctg    1320
   tacgacaaag tcagccaggc gttcaccgtc ttcctgccag tacgctccgt tggcgtaatg    1380
   ggcgatggtc gtaagtacga ttgggtggtc tccctgcgtg ctgtcgaaac catcgacttt    1440
   atgactgcgc actgggcgca tctgccgtat gacttcctgg gtcgtgtttc caaccgcatc    1500
   atcaatgaag tcaacgggat ttcccgtgtg gtgtatgaca tcagcggtaa accaccggct    1560
   accattgagt gggaataa                                                  1578
<210> 37
   <211> 624
   <212> DNA
   <213> Salmonella Paratyphi A 9150
<220>
   <221> misc_feature
   <223> clpP
<400> 37
   atgtcataca gcggagaacg agataatttg gcccctcata tggcgctggt gccgatggtc      60
   attgaacaga cctcacgcgg tgagcgctct tttgatatct attctcgtct acttaaggaa     120
   cgcgtcatat ttctgaccgg ccaggtcgaa gaccatatgg ctaacctgat cgtggcgcag     180
   atgctgttcc tggaagcgga aaacccggaa aaagatatct atctgtacat taattctcct     240
   ggcggcgtaa ttactgcggg gatctccatc tatgacacca tgcagtttat taagccagac     300
   gtcagcacca tttgtatggg acaggcggcc tctatggggg cgtttctgct gactgccggg     360
   gcgaaaggca aacgtttctg cttgccgaac tctcgcgtca tgatccacca gccgctgggc     420
   ggctaccagg gccaggcgac ggatattgaa attcacgccc gcgaaatttt gaaagtaaaa     480
   gggcgcatga atgaacttat ggcgcatcat acgggtcaat ctcttgagca gattgaacgt     540
   gatactgagc gcgatcgctt cctctccgcg cctgaagcgg tagagtacgg tttggttgac     600
   tcaattttga cccatcgtaa ttga                                            624
<210> 38
   <211> 1272
   <212> DNA
   <213> Salmonella Paratyphi A 9150
<220>
   <221> misc_feature
   <223> clpX
<400> 38
   atgacagata aacgcaaaga tggctcgggc aaattgttgt actgctcttt ttgcggcaaa 60
   agccagcatg aagtgcgcaa gctgattgcc ggtccatccg tgtatatctg cgacgaatgc 120
   gtcgatttat gtaacgacat tattcgcgaa gaaattaaag aagttgctcc gcaccgtgaa 180
   cgtagtgcgc tgccgacgcc gcatgaaatt cgtacccacc tggacgatta cgttatcggc 240
   caggagcagg cgaaaaaagt gctggcggtg gcggtctata accactacaa gcgtctgcgt 300
   aacggtgata ccagcaatgg cgtcgagtta ggcaaaagca acattctgct gattggaccg 360
   accggttccg gtaaaacgct gctggcggaa acgctggcgc gcttgctgga tgtgccgttc 420
   actatggcgg atgcgaccac gctgaccgaa gcgggttacg tgggtgaaga cgtcgagaat 480
   atcattcaga aactgttgca gaaatgcgac tacgacgtgc aaaaagcgca gcgtgggatt 540
   gtctacattg atgaaatcga taagatttcg cgtaaatcag acaatccgtc cattacccgc 600
   gatgtttccg gcgaaggcgt acagcaggcg ttgctgaaac tgatcgaagg caccgtcgcc 660
   gcggttccac cgcagggcgg tcgcaaacat ccgcagcagg agttcttaca ggtagatacc 720
   tctaagattc tgtttatctg cggcggcgcg tttgctggtc tggataaagt gatcgctaac 780
   cgtgttgaaa ccggctccgg cattggtttt ggcgcgacgg tgaaagcgaa gtccgacaaa 840
   gccagcgaag gcgagctgtt gtcgcaggtt gaaccggaag atttgatcaa atttggtctg 900
   attcctgagt ttatcggtcg tctgccagtg gtggcgacgc tgaacgaact cagcgaagaa 960
   gcgctgattc aaatcctgaa agagccgaaa aatgcgctga ccaagcagta tcaggcgctg 1020
   tttaacctgg aaggcgtcga tctggaattc cgtgacgaag cgctggacgc tatcgccagg 1080
   aaagcaatgg cgcgtaaaac cggtgcccgt ggtctgcgtt ctatcgtcga agcggcgctg 1140
   ctggatacca tgtacgattt gccatctatg gaagacgtcg aaaaagtggt gatcgacgag 1200
   tccgttattg ccggtcagag taagccgttg ctgatttacg gcaaaccgga agcgcaggct 1260
   tctggcgaat aa 1272
<210> 39
   <211> 1272
   <212> DNA
   <213> Salmonella Typhi Ty2
<220>
   <221> misc_feature
   <223> clpX gene
<400> 39
   atgacagata aacgcaaaga tggctcgggc aaattgttgt actgctcttt ttgcggcaaa      60
   agccagcatg aagtgcgcaa gctgattgcc ggtccatccg tgtatatctg cgacgaatgc     120
   gtcgatttat gtaacgacat tattcgcgaa gaaattaaag aagttgctcc gcaccgtgaa     180
   cgtagtgcgc tgccgacgcc gcatgaaatt cgtactcacc tggacgatta cgttatcggc     240
   caggagcagg cgaaaaaagt gctggcggtg gcggtctata accactacaa gcgtctgcgt     300
   aacggcgata ccagcaatgg cgtcgagtta ggcaaaagca acattctgct gactggaccg     360
   accggttccg gtaaaacgct gctggcggaa acgctggcgc gcttgctgga tgtgccgttc     420
   actatggcgg atgcgaccac gctgaccgaa gcgggttacg tgggggaaga cgtcgagaat     480
   atcattcaga aactgttgca gaaatgcgac tacgacgtgc aaaaagcgca gcgtgggatt     540
   gtctacattg atgaaatcga taagatttcg cgtaaatcag acaatccgtc cattacccgc     600
   gatgtttccg gcgaaggcgt acagcaggcg ttgctgaaac tgatcgaagg caccgtcgcc     660
   gcggttccac cgcagggcgg tcgcaaacat ccgcagcagg agttcttaca ggtagatacc     720
   tctaagattc tgtttatctg cggcggcgcg tttgccggtc tggataaagt gatcgctaac     780
   cgtgttgaaa ccggctccgg cattggtttt ggcgcgacgg tgaaagcgaa gtccgacaaa     840
   gccagtgaag gcgagctgtt gtcgcaggtt gaaccggaag atttgatcaa atttggtctg     900
   attcctgagt ttatcggtcg tctgccagtg gtggcgacgc tgaacgaact cagcgaagaa     960
   gcgctggttc aaatcctgaa agagccgaaa aatgcgctga ccaagcagta tcaggcgctg    1020
   tttaacctgg aaggcgtcga tctggaattc cgtgacgaag cgctgaacgc tatcgccagg    1080
   aaagcgatgg cgcgtaaaac tggtgcccgt ggcctgcgtt ctatcgtcga agcggcgctg    1140
   ctggatacca tgtacgattt gccatctatg gaagacgtcg aaaaagtggt gattgacgag    1200
   tccgttattg ccggtcagag taagccgttg ctgatttacg gcaaaccgga agcgcaggct    1260
   tctggcgaat aa                                                        1272
<210> 40
   <211> 624
   <212> DNA
   <213> Salmonella Typhi Ty2
<220>
   <221> misc_feature
   <223> clpP gene
<400> 40
   atgtcataca gcggagaacg agataatttg gcccctcata tggcgctggt gccgatggtc 60
   attgaacaga cctcacgcgg tgagcgctct tttgatatct attctcgtct acttaaggaa 120
   cgcgtcatat ttctgaccgg ccaggtcgaa gaccatatgg ctaacctgat cgtggcgcag 180
   atgctgttcc tggaagcgga aaacccggaa aaagatatct atctgtacat taattctcct 240
   ggcggcgtaa ttactgcggg gatgtccatc tatgacacca tgcagtttat taagccagac 300
   gtcagcacca tttgtatggg acaggcggcc tctatggggg cgtttctgct gactgccggg 360
   gcgaaaggca aacgtttctg cttgccgaac tctcgcgtca tgatccacca gccgctgggc 420
   ggctatcagg gccaggcgac ggatattgaa attcacgccc gcgaaatttt gaaagtaaaa 480
   gggcgcatga atgaacttat ggcgcatcat acgggtcaat ctcttgagca gattgaacgt 540
   gatactgagc gcgatcgctt cctctccgcg cctgaagcgg tagagtacgg tttggttgac 600
   tcaattttga cccatcgtaa ttga 624
<210> 41
   <211> 32
   <212> DNA
   <213> Artificial Sequence
<220>
   <223> chemically-synthesized oligonucleotide primer
<400> 41
   tcacgcacac gctgcaggtt gttgttgatt tc 32
<210> 42
   <211> 47
   <212> DNA
   <213> Artificial Sequence
<220>
   <223> chemically-synthesized oligonucleotide primer
<400> 42
   gaacttcgaa gcagctccag cacctaatga tgaaattgaa gccatgc 47
<210> 43
   <211> 21
   <212> DNA
   <213> Artificial Sequence
<220>
   <223> chemically-synthesized oligonucleotide primer
<400> 43
   gctggagctg cttcgaagtt c 21
<210> 44
   <211> 33
   <212> DNA
   <213> Artificial Sequence
<220>
   <223> chemically-synthesized oligonucleotide primer
<400> 44
   ctcgagttcc ggggatccgt cgacctgcag ttc 33
<210> 45
   <211> 34
   <212> DNA
   <213> Artificial Sequence
<220>
   <223> chemically-synthesized oligonucleotide primer
<400> 45
   ggatccgcta tgaacaagtc ctgataacag aggt 34
<210> 46
   <211> 46
   <212> DNA
   <213> Artificial Sequence
<220>
   <223> chemically-synthesized oligonucleotide primer
<400> 46
   ctcgagttaa cgagactcct ggaaagatgc tttcggtgaa atctgc 46
<210> 47
   <211> 2363
   <212> DNA
   <213> Salmonella enterica serovar Typhimurium
<400> 47
   tcacgcacac gctgcaggtt gttgttgatt tcgttcagcg cgccttcagt ggtctgcgca 60
   atggagatac cgtcgttagc gttacgggaa gcctgagtca gacctttgat gttcgcggta 120
   aaacggttag caatcgcctg acctgccgca tcgtctttcg cgctgttgat acgcagaccg 180
   gaagacagac gctcgatagc ggtgcccaga gcggactggg atttgttcag gttattctgg 240
   gtcaacagcg acaggctgtt tgtattaatg acttgtgcca tgatcttttc cttatcaatt 300
   acaacttgat gttattgggc tgttgcccac ggtttctcac cgtaaccctt gtatcggcac 360
   ctgaatttcg aactttagaa aatttttcac ttcccccgat ctttttctta ggcggcgaaa 420
   tagccgcttt atgcatcatt attccgcgca ttatttttgc aaaattatca ttaaactttg 480
   cctccagatt gccgataacg cgcttaacta ctgtttgcaa tcaaaaagga agaaggcatg 540
   gcttcaattt catcattagg tgtgggatca aacttaccgt tagaccagtt gttgacagac 600
   ctgacaaaga acgaaaaagg acgcttaacg ccaattacca aacagcagag cgcgaattcg 660
   gcaaagctaa ccgcctatgg cacattgaaa agcgcattag aaaaattcca gacggcaaat 720
   accgcgttaa ataaagcgga tttatttaag tctaccgtgg cgtccagcac cactgaagat 780
   ctcaaagtca gtactaccgc tggcgctgcc gcagggactt ataagattaa cgtaacccag 840
   cttgccgccg cacagtcgct ggcgacaaaa accaccttcg cgaccaccaa agagcagttg 900
   ggcgatacgt cggtcacgtc ccggacaatt aaaattgaac agccgggacg taaagagccg 960
   ctggaaatta agctggataa aggcgacacc tccatggagg cgatccgtga cgccatcaat 1020
   gacgccgaca gcggtatcgc cgccagtatc gttaaggtca aagagaacga attccagttg 1080
   gtgcttaccg ccaatagcgg taccgacaat acgatgaaga tcacggtgga aggcgataca 1140
   aaacttaacg atctactcgc ttatgacagc accaccaata ccggcaatat gcaagagctg 1200
   gtgaaagcag aaaacgcgaa gctgaacgta aacggcatcg acattgagcg tcagagcaat 1260
   accgtaaccg acgcccctca gggaattacg ctcaccctga ccaagaaagt gaccgacgcg 1320
   accgtgacgg taacgaaaga tgataccaag gcgaaagagg cgattaaatc ctgggtggat 1380
   gcctataact cgctggtgga tacctttagc tcgttaacca aatataccgc cgttgagccg 1440
   ggcgaagaag ccagcgataa aaacggcgcg ctgttaggcg atagtgtggt tcgtactatc 1500
   cagaccggga ttcgggcaca atttgccaat agcggcagta attctgcgtt caaaacaatg 1560
   gcggaaattg gcatcaccca ggatgggact tccggcaaac tgaagattga tgatgataag 1620
   ctgaccaaag tactgaaaga taacacagcc gcagcgcgtg agctgctggt aggcgatggt 1680
   aaagaaacgg gtatcaccac caaaattgcc accgaagtga aaagttatct ggcggatgac 1740
   ggcattattg ataatgcgca ggacaacgtt aacgccacgc tgaaaagcct gacaaaacag 1800
   tacctgtccg ttagcaacag catcgatgaa accgttgccc gttacaaggc ccagtttacc 1860
   caactggata ccatgatgag taagctgaat aacaccagta gttatttgac ccagcaattt 1920
   acagctatga acaagtcctg ataacagagg tcaccatgta caccgcgagc ggtatcaaag 1980
   cttatgcgca agtcagcgtg gaaagcgccg tgatgagcgc cagcccgcat cagttgattg 2040
   aaatgttgtt tgatggcgcg aatagcgctc tggtgcgcgc tcgcctgttt ttagaacaag 2100
   gcgatgttgt cgcgaaaggt gaagcgttaa gcaaagccat caatattatc gataacgggc 2160
   tgaaagccgg cctcgatcag gaaaaaggcg gtgagattgc gacgaatctt tccgagctat 2220
   acgactatat gattcgccgt ttactgcagg ctaatttgcg taacgacgct caggccatcg 2280
   aagaagtgga agggttactc agcaatattg cagaagcctg gaagcagatt tcaccgaaag 2340
   catctttcca ggagtctcgt taa 2363
<210> 48
   <211> 2363
   <212> DNA
   <213> Salmonella enterica serovar Paratyphi A
<400> 48
   tcacgcacac gctgcaggtt gttgttgatt tcgttcagcg cgccttcagt ggtctgcgca 60
   atggagatac cgtcgttagc gttacgggaa gcctgagtca gacctttgat gttcgcggtg 120
   aaacggttag caattgcctg acctgccgca tcgtctttcg cgctgttgat acgcagaccg 180
   gaagacagac gctcgatagc ggtgcccaga gcggactggg atttgttcag gttattctgg 240
   gtcaacagcg acaggctgtt tgtattaatg acttgtgcca tgatcttttc cttatcaatt 300
   acaacttgat gttattgggc tgttgcccac ggtttctcac cgtaaccctt gtatcggcac 360
   ctgaatttcg aactttagaa aatttttcac ttcccccgat ctttttctta ggcggcgaaa 420
   tagccgcttt atgcatcatt attccgcgca ttatttttgc aaaattatca ttaaactttg 480
   cctccagatt gccgataacg cgcttaacta ctgtttgcaa tcaaaaagga agaaggcatg 540
   gcttcaattt catcattagg tgtgggatca aacttaccgt tagaccagtt gttgacagac 600
   ctgacaaaga acgaaaaagg acgcttaacg ccaattacca aacagcagag cgcgaattcg 660
   gcaaagctaa ccgcctatgg cacattgaaa agcgcattag aaaaattcca gacggcaaat 720
   accgcgttaa ataaagcgga tttatttaag tctaccgtgg cgtccagcac gacagaggac 780
   ctcaaagtca gtactaccgc aggcgctgcc gcagggactt ataagattag cgtaacccag 840
   cttgccgccg cgcagtcgct ggcgacaaaa accaccttcg caaccaccaa agagcagttg 900
   ggcgatacgt cggtcacgtc ccggacaatt aaaattgaac agccgggacg taaagagccg 960
   ctggaaatta agctggataa aggcgacacc tccatggagg cgatccgtga cgccatcaat 1020
   gacgccgaca gcggtatcgc cgccagtatc gttaaggtca aagagaacga attccagttg 1080
   gtgcttaccg ccaatagcgg taccgacaat acgatgaaga tcacggtgga aggcgataca 1140
   aaacttaacg atctactcgc ttatgacagc accaccaata ccggcaatat gcaagagctg 1200
   gtgaaagcag aaaacgcgaa gctgaacgta aacggcatcg acattgagcg tcagagcaat 1260
   accgtaaccg acgcccctca gggaattacg ctcaccctga cgaagaaagt gaccgacgcg 1320
   accgtgacgg taacgaaaga tgataccaag gcgaaagagg cgattaaatc ctgggtggat 1380
   gcctataact cgctggtgga tacctttagc tcgttaacca aatataccgc cgttgagccg 1440
   ggcgaagaag ccagcgataa aaacggcgcg ctgttaggcg atagtgtggt tcgtactatc 1500
   cagaccggga ttcgggcaca atttgccaat agcggcagta attctgcgtt caaaacaatg 1560
   gcggaaattg gcatcaccca ggatgggact tccggcaaac tgaagattga tgatgataag 1620
   ctgaccaagg tactgaaaga taacacagcc gcagcgcgtg agctgctggt aggcgatggt 1680
   aaagaaacgg gtatcaccac caaaattgcc accgaagtga aaagttatct ggcggatgac 1740
   ggcattattg ataatgcgca ggacaacgtt aacgccacgc tgaaaagcct gacaaaacag 1800
   tacctgtccg ttagcaacag catcgatgaa accgttgccc gttacaaggc ccagtttacc 1860
   caactggata ctatgatgag taagctgaat aacaccagta gttatttgac ccagcaattt 1920
   acagctatga acaagtcctg ataacagagg tcaccatgta caccgcgagc ggtatcaaag 1980
   cttatgcgca agtcagcgtg gaaagcgccg tgatgagcgc cagcccgcat cagttgattg 2040
   aaatgttgtt tgatggcgcg aatagcgctc tggtgcgcgc tcgcctgttt ttagaacaag 2100
   gcgatgttgt cgcgaaaggt gaagcgttaa gcaaagccat caatattatc gataacgggc 2160
   tgaaagccgg cctcgatcag gaaaaaggcg gtgagattgc gacgaatctt tccgagctat 2220
   acgactatat gattcgccgt ttactgcagg ctaatttgcg taacgacgct caggccatcg 2280
   aagaagtgga agggttactc agcaatattg cagaagcctg gaagcagatt tcaccgaaag 2340
   catctttcca ggagtctcgt taa 2363
<210> 49
   <211> 2363
   <212> DNA
   <213> Salmonella enterica serovar Typhi strain Ty2
<400> 49
   tcacgcacac gctgcaggtt gttgttgatt tcgttcagcg cgccttcagt ggtctgcgca      60
   atggagatac cgtcgttagc gttacgggaa gcctgagtca gacctttgat gttcgcggta     120
   aaacggttag caatcgcctg tcctgccgca tcgtctttcg cgctgttgat acgcagaccg     180
   gaagacaaac gctcgatagc agtgcccagt gcggactggg atttgttcag gttattctgg     240
   gtcaacagcg acaggctgtt tgtattaatg acttgtgcca tgatcttttc cttatcaatt     300
   acaacttgat gttattgggc tgttgcccac ggtttctcac cgtaaccctt gtatcggcac     360
   ctgaatttcg aactttagaa aatttttcac ttcccccgat ctttttctta ggcggcgaaa     420
   tagccgcttt atgcatcatt attccgcgca ttatttttgc aaaattatca ttaaactttg     480
   cctccagatt gccgataacg cgcttaacta ctgtttgcaa tcaaaaagga agaaggcatg     540
   gcttcaattt catcattagg tgtgggatca aacttaccgt tagaccagtt gttgacagac     600
   ctgacaaaga acgaaaaagg acgcttaacg ccaattacca aacagcagag cgcgaattcg     660
   gcaaagctaa ccgcctatgg cacattgaaa agcgcattag aaaaattcca gacggcaaat     720
   accgcgttaa ataaagcgga tttatttaag tctaccgtgg cgtccagcac gacagaggac     780
   ctcaaagtca gtactaccgc aggcgctgcc gcagggactt ataagattag cgtaacccag     840
   cttgccgccg cgcagtcgct ggcgacaaaa accaccttcg caaccaccaa agagcagttg     900
   ggcgatacgt cggtcacatc ccggacaatt aaaattgaac agccgggacg taaagagccg     960
   ctggaaatta agctggataa aggcgacacc tccatggagg cgatccgtga cgccattaat    1020
   gacgccgaca gcggtatcgc cgccagtatc gttaaggtca aagagaacga attccagttg 1080
   gtgcttaccg ccaatagcgg taccgacaat acgatgaaga tcacggtgga aggcgataca 1140
   aaacttaacg atctactcgc ttatgacagc accaccaata ccggcaatat gcaagagctg 1200
   gtgaaagcag aaaacgcgaa gctgaacgta aacggcatcg acattgagcg tcagagcaat 1260
   accgtaaccg acgcccctca gggaattaca ctcaccctga ccaagaaagt gaccgacgcg 1320
   accgtgacgg tgacgaaaga tgataccaag gcgaaagagg cgattaaatc ctgggtggat 1380
   gcctataact cgctggtgga tacttttagc tcattaacta aatataccgc cgttgagccg 1440
   ggcgaagaag ccagcgataa aaacggcgcg ctgttaggcg atagtgtggt tcgtgctatc 1500
   cagaccggga ttcgggcaca atttgccaat agcggcagta attctgcgtt caaaacaatg 1560
   gcggaaattg gcatcaccca ggatgggact tccggcaaac tgaagattga cgatgataag 1620
   ctgaccaagg tactgaaaga taacacggcc gcagcgcgtg agctgctggt aggcgatggt 1680
   aaagaaacgg gtatcaccac caaaattgcc accgaagtga aaagttatct ggcggatgac 1740
   ggcattattg ataatgcgca ggacaacgtt aacgccacgc tgaaaagcct gacaaaacag 1800
   tacctgtccg ttagcaacag catcgatgaa accgttgccc gttacaaggc ccagtttacc 1860
   caactggata ccatgatgag taagctgaat aacaccagta gttatttgac ccagcaattt 1920
   acagctatga acaagtcctg ataacagagg tcaccatgta caccgcgagc ggtatcaaag 1980
   cttatgcgca agtcagcgtg gaaagcgccg tgatgagcgc cagcccgcat cagttgattg 2040
   aaatgttgtt tgatggcgcg aatagcgctc tggtgcgcgc tcgcctgttt ttagaacaag 2100
   gcgatgttgt cgcgaaaggt gaagcgttaa gcaaagccat caatattatc gataacgggc 2160
   tgaaagccgg cctcgatcag gaaaaaggcg gtgagattgc gacgaatctt tccgagctat 2220
   acgactatat gattcgccgt ttactgcagg ctaatttgcg taacgacgct caggccatcg 2280
   aagaagtgga aaggttactc agcaatattg cagaagcctg gaagcagatt tcaccgaaag 2340
   catctttcca ggagtctcgt taa 2363
<210> 50
   <211> 2365
   <212> DNA
   <213> Salmonella enterica serovar Enteritidis
<400> 50
   tcacgcacac gctgcaggtt gttgttgatt tcattcagcg caccttcagt ggtctgcgca    60
   atagaaatgc cgtcgttagc gttacgggaa gcctgagtca gacctttgat attagaagtg   120
   aagcggttag caatcgcctg gcctgccgca tcgtctttcg cgctgttgat acgcagacca   180
   gaggacagac gctcaatagc ggaactcagt gaggactgag atttgttcag gttattctgg   240
   gtcaacagcg acaggctgtt tgtattaatg acttgtgcca tgatcttttc cttatcaatt   300
   acaacttgat gttattgggc tgttgcccac ggtttctcac cgtaaccctt gtatcggcac   360
   ctgaatttcg aactttagaa aatttttcac ttcccccgat ctttttctta gcctgcgaaa 420
   tagccgcttt atgcatcatt attccacgca ttatttttgc aaaattatca ttaaactttg 480
   cttccagatt gccgataacg cgtttaacta ctgtttgcaa tcgaaaggaa gatggcatgg 540
   cttcaatttc atcattaggt gtagggtcaa acttacctct ggattcactg ctgactaaac 600
   tgaccaacgc tgaaaaagga cgcttaacgc cgatcacaca gcagcagagt gctaatacgg 660
   cccgtctaac ggcatacggt actttaaaaa gtgcactgga gaagtttcaa acagcaaaca 720
   cggcgttaaa taaagccgat ctgtttaaaa gtacgaatgt caccagcagt acagaagacc 780
   tgaaagtctc gacggaagct ggggccgcac ctggaactta tgtggttagc gtaactcagt 840
   tagcacaagc acaatctttg agtacagcaa ccaaaattac atctaccaaa gaagtgctgg 900
   gagataccac atctgacagc cgtaccataa aaattgaaca gaaaggccgt aaagaaccac 960
   ttgaaatcaa gctcactaaa gatcaaacct ctttagaggg tatccgtgac gccattaatg 1020
   atgctgacag tggtatttcc gccagtatcg ttaaagttaa agaaggcgat tatcagcttg 1080
   tactgaccgc agatagtggc acggataatc aaatgactat ctctgtggaa ggcgatagca 1140
   aactcagcga tctgttgtcc tatgatagta gtactggcac gggcaaaatg aagcaactgg 1200
   ttgctgcaga taatgctttg ttaaccgtta acggcattga tattgagcga ccgagtaata 1260
   aaatcactga cgctccacaa ggcgtgacgc ttgaactaac caaagaagta aaagatgccc 1320
   gtattaccgt cacaaaagat aatgaaaagg cgaccgaagc cgtcaaaggt tgggttgatg 1380
   cctacaactc actgcttgat acctttagtt cattaacaaa atatacagag gttgatccag 1440
   gggctgaaga acaggacaaa aacaacggtg cactacttgg agataccgtg gtgcgaacga 1500
   ttcaaactgg aatccgcgct cagttcgcta atggtgcaag tacaggtaca tttaagaccc 1560
   tgaatgaaat tggtattact tctgatggta ccaccggaaa actaaaaatt gatgatacca 1620
   agcttaaaaa agcgctggat gaaaataccg cttctgtacg tgagctgctg gtaggtgatg 1680
   gtaaagaaac ggggatcacc accaaaattg ccaccgaagt gaaaagttat ctggccgatg 1740
   acggcattat tgacagcgcc caggacagta ttaacgccac gctgaaaaag ctgactaagc 1800
   aatatctgac cgtcagcagt agcattgacg acaccgttgc ccgttacaag gcccagttta 1860
   cccaactgga taccatgatg agtaagctga ataacaccag tacttatttg acccagcaat 1920
   ttaatgctat gaacaagtcc tgataacaga ggttaccatg tacaccgcga gcggtatcaa 1980
   agcttatgcg caagtcagcg tggaaagcgc cgtgatgagc gccagcccgc atcagttgat 2040
   tgaaatgttg tttgatggcg cgaatagcgc tctggtgcgc gctcgcctgt ttttagaaca 2100
   aggcgatgtt gtcgcgaaag gtgaagcgtt aagcaaagcc atcaatatta tcgataacgg 2160
   gctgaaagcc ggcctcgatc aggaaaaagg cggtgagatt gcgacgaatc tttccgagct 2220
   atacgactat atgattcgcc gtttactgca ggctaatttg cgtaacgacg ctcaggccat 2280
   cgaagaagtg gaagggttac tcagcaatat tgcagaagcc tggaagcaga tttcaccgaa 2340
   agcatctttc caggagtctc gttaa                                       2365